# EUROPEAN PATENT APPLICATION

(11) **EP 4 524 156 A1**
(43) Date of publication of application: **19.03.2025**
(21) Application number: 23802753.6
(22) Date of filing: 05.05.2023
(51) Int. Cl.: C07K 16/18, C07K 16/46, C07K 19/00, C12N 15/13, C12N 15/62, A61K 39/395, A61P 9/00

(54) **FUSION PROTEIN OF RELAXIN OR ANALOGUE AND MEDICAL USE THEREOF**

(30) Priority: 07.05.2022 CN 202210491720
(71) Applicant: Beijing Tuo Jie Biopharmaceutical Co. Ltd., Beijing 102206 (CN)
(72) Inventor: DU, Yanping, Beijing 102206 (CN); WANG, Lei, Beijing 102206 (CN); LIU, Xiao, Beijing 102206 (CN); GAO, Hong, Beijing 102206 (CN); SHEN, Chenxi, Beijing 102206 (CN); ZHANG, Linshuang, Beijing 102206 (CN); JIANG, Peng, Beijing 102206 (CN)
(74) Representative: Regimbeau
(86) International application number: PCT/CN2023/092214
(87) International publication number: WO 2023/216981

(57) **Abstract**

The present disclosure relates to a fusion protein of relaxin or an analogue and the medical use thereof. Specifically, the present disclosure relates to a fusion protein comprising relaxin or an analogue thereof and a serum albumin binding domain, and the medical use thereof for treating diseases (for example, heart failure). Moreover, the present disclosure relates to a serum albumin binding molecule, which comprises an immunoglobulin single variable domain (for example, VHH) capable of binding to serum albumin, has good plasma stability, and can be used for prolonging the plasma half-life of a drug.

## Description

The present disclosure claims priority to the Chinese Patent Application (Application No. CN202210491720.7) filed on May 7, 2022.

### TECHNICAL FIELD

The present disclosure belongs to the field of biomedicine, and particularly relates to a fusion protein comprising relaxin or an analog thereof and a serum albumin-binding domain, and medical use thereof in the treatment of a disease (e.g., heart failure). Moreover, the present disclosure relates to a serum albumin-binding molecule, which comprises an immunoglobulin single variable domain (e.g., VHH) capable of binding to serum albumin, has good plasma stability, and can be used for extending the plasma half-life of a drug.

### BACKGROUND

Relaxin, an important hormone during pregnancy, was first discovered in 1926 by Frederick Hisaw. It is a polypeptide hormone, belonging to the insulin superfamily. In humans, relaxin includes 7 polypeptide members, including relaxin-1 (RLN-1 or H1), relaxin-2 (RLN-2 or H2), and relaxin-3 (RLN-3 or H3). These relaxins have low homology in their primary sequences but possess similar tertiary structures. Among them, relaxin-2 is the major relaxin in the human circulation and is encoded by the RLN2 gene. The relaxin is expressed in the form of a prohormone (with the A and B chains linearly linked by a C-peptide), which undergoes endonuclease hydrolysis *in vivo* to remove C-peptide, thereby releasing mature relaxin. Natural mature relaxin-2 contains two polypeptide chains, A and B, which are covalently linked by two interchain disulfide bonds to form a heterodimer, and there is also a pair of disulfide bonds within the A chain (Schwabe, et al., Science. 1977, 197, 914-915).

Relaxin-2 is a pleiotropic hormone whose signaling is primarily achieved by the receptor RXFP1 (also known as LGR7). RXFP1 is a G protein-coupled receptor (GPCR) that is rich in leucine repeat units. RXFP2 (also known as LGR8) also binds to relaxin-2. The binding of relaxin-2 to the receptor can activate a variety of cascade signals, including activation of the cAMP and phosphoinositide 3-kinase pathways, activation of the nitric oxide signaling pathway, and the like. The binding of relaxin-2 to the receptor can also inhibit phosphorylation of Smad2/3, thereby inhibiting the production of extracellular matrix and collagen. Research shows that relaxin-2 has the physiological effects of promoting vasodilation, resisting inflammation, decreasing vascular resistance and increasing arterial compliance, promoting angiogenesis and remodeling, regulating extracellular matrix, resisting fibrosis, and the like, and thus exerts beneficial effects on heart failure and myocardial fibrosis (Chiara Sassoli, et al., Current Molecular Medicine 2022, 22, 196-208).

The half-life (t_{1/2}) of relaxin-2 is very short. The half-life (t_{1/2}) of natural relaxin-2 *in vivo* is only a few minutes, which poses a challenge for the use of relaxin as a therapeutic agent. The natural recombinant relaxin-2 (serelaxin) that is already on the market requires continuous intravenous infusion in clinical treatment, which is inconvenient for patients and also demonstrates short-lived efficacy. Researchers have engineered relaxin-2 to increase its half-life (t_{1/2}), for example, by introducing PEG, fatty acid chains, or the like through chemical coupling, or by fusing it to half-life extending proteins, polypeptides, Fcs, or the like through recombinant methods.

Albumin is the most abundant protein in plasma, accounting for 40-60% of plasma proteins. Albumin can bind to many endogenous and exogenous ligands, making it a natural "multifunctional carrier" *in vivo.* Albumin is similar to immunoglobulin G (IgG) in that it has a relatively long retention time *in vivo.* It has been reported that the half-life of albumin in the human body is approximately 19-21 days (Berson, S.A. et al., J. Clin. Invest. 1953, 32, 746-768). This is primarily because albumin has a molecular weight of 66.5 KD, and its molecular hydrodynamic radius exceeds the glomerular filtration threshold of 3.5-6 nm (Lin, J. H., Curr. Drug Metab. 2009, 10, 661-691). Albumin can bind to FcRn, and this binding is characterized by pH dependency. After entering an endosome through endocytosis, the albumin and the FcRn are bound tightly at the acidic pH (pH 5.5 or 6.0) of the endosome, so that the albumin is protected from lysosomal degradation. As the FcRn is transported to the cell surface, the albumin dissociates from the FcRn under neutral physiological pH conditions, allowing for its recycling. This mechanism is similar to the recycling of IgG. Moreover, albumin and IgG bind to two separate sites on FcRn, which do not interfere with each other.

The present disclosure provides a novel structured single-domain antibody binding to serum albumin, which is capable of significantly increasing the *in vivo* half-life of an active pharmaceutical ingredient. For example, when the single-domain antibody binding to serum albumin of the present disclosure is fused to relaxin or an analog thereof as an active ingredient to form a fusion protein, the *in vivo* half-life of the relaxin or the analog thereof can be significantly increased. The fusion protein provided by the present disclosure has high activity, high stability, convenient production, and excellent potential for clinical application.

### SUMMARY

The present disclosure provides a serum albumin-binding molecule, which comprises an immunoglobulin single variable domain specifically binding to serum albumin, has good plasma stability, and can be used for extending the plasma half-life of a drug.

### Serum Albumin-Binding Molecule

The present disclosure provides a serum albumin-binding molecule.

In some embodiments, the serum albumin-binding molecule comprises an immunoglobulin single variable domain, the immunoglobulin single variable domain comprising a CDR1, a CDR2, and a CDR3 as described in any one of the following 1)-5):
1) a CDR1, a CDR2, and a CDR3 from the amino acid sequence set forth in any one of SEQ ID NOs: 6 and 18-27;
2) a CDR1, a CDR2, and a CDR3 from the amino acid sequence set forth in SEQ ID NO: 7;
3) a CDR1, a CDR2, and a CDR3 set forth in SEQ ID NOs: 8, 9, and 10, respectively;
4) a CDR1, a CDR2, and a CDR3 set forth in SEQ ID NOs: 11, 12, and 13, respectively; and
5) a CDR1 of X₁SCX₂G (SEQ ID NO: 64), wherein X₁ is T or N, and X₂ is M or L;
   a CDR2 of X₃IYX₄VGGSTFYX₅X₆SVKG (SEQ ID NO: 58), wherein X₃ is S or T, X₄ is T or M, X₅ is T or A, and X₆ is N or D; and
   a CDR3 of GSPARCX₇X₈RDPTTFX₉Y (SEQ ID NO: 59), wherein X₇ is R or L, X₈ is L or R, and X₉ is D or N;
   wherein one or more of the X₁ to X₉ described above may be a conservative amino acid mutation of an optional amino acid thereof.

The CDRs described above are defined according to the Kabat, IMGT, Chothia, AbM, or Contact numbering scheme, for example, the CDRs are defined according to the Kabat numbering scheme.

In some embodiments, the serum albumin-binding molecule comprises an immunoglobulin single variable domain, the immunoglobulin single variable domain comprising any one of or a combination of any several (e.g., 2 or 3) of the CDR1s, the CDR2s, and the CDR3s from 1)-5) described above.

In some embodiments, the serum albumin-binding molecule comprises an immunoglobulin single variable domain, wherein the immunoglobulin single variable domain comprises a CDR1 having 0, 1, 2, 3, 4, or 5 amino acid mutations compared to any one of the aforementioned CDR1s; and/or the immunoglobulin single variable domain comprises a CDR2 having 0, 1, 2, 3, 4, or 5 amino acid mutations compared to any one of the aforementioned CDR2s; and/or the immunoglobulin single variable domain comprises a CDR3 having 0, 1, 2, 3, 4, or 5 amino acid mutations compared to any one of the aforementioned CDR3s. In some specific embodiments, the amino acid mutations in the CDR1, the CDR2, and/or the CDR3 are conservative substitutions.

In some embodiments, the aforementioned immunoglobulin single variable domain is camelid-derived.

In some embodiments, the aforementioned immunoglobulin single variable domain is engineered by any one of the following: humanization, affinity maturation, T cell epitope removal, reduction of antibody deamidation, reduction of antibody aggregation, reduction of antibody isomerization, or a combination thereof.

In some embodiments, a framework region in the aforementioned humanized immunoglobulin single variable domain is derived from the human heavy chain variable region germline gene IGHV3-23 or IGVH3-66.

In some specific embodiments, the aforementioned humanized immunoglobulin single variable domain comprises an amino acid mutation at at least one of the following positions: positions 20, 23, 27, 29, 30, 37, 44, 45, 47, 49, 74, 78, 83, and 84 (positions based on the EU numbering scheme);
for example, the humanized immunoglobulin single variable domain comprises at least one amino acid mutation selected from the group consisting of the following: 20V, 23V, 27N, 29Y, 30L, 37F, 44E, 45R, 47G, 49A, 74A, 78V, 83Q, and 84P;
for example, the humanized immunoglobulin single variable domain comprises a combination of amino acid mutations selected from any one of the following:
   F27N, F29Y, S30L, V37F, G44E, L45R, and W47G;
   L20V, F27N, F29Y, S30L, V37F, G44E, L45R, and W47G;
   A23V, F27N, F29Y, S30L, V37F, G44E, L45R, and W47G;
   F27N, F29Y, S30L, V37F, G44E, L45R, W47G, and S74A;
   F27N, F29Y, S30L, V37F, G44E, L45R, W47G, and L78V;
   F27N, F29Y, S30L, V37F, G44E, L45R, W47G, and S49A;
   F27N, F29Y, S30L, V37F, G44E, L45R, W47G, R83Q, and A84P;
   F27N, F29Y, S30L, V37F, G44E, L45R, W47G, and A84P;
   S30L, V37F, G44E, L45R, W47G, and A84P; or
   S30L, V37F, G44E, L45R, and W47G.

In some embodiments, the serum albumin-binding molecule provided by the present disclosure comprises an immunoglobulin single variable domain, the immunoglobulin single variable domain comprising or being any one of SEQ ID NO: 6 or 7 or an amino acid sequence having at least 80% or at least 90% sequence identity thereto. In some embodiments, the immunoglobulin single variable domain is camelid-derived.

In some embodiments, the serum albumin-binding molecule provided by the present disclosure comprises an immunoglobulin single variable domain, the immunoglobulin single variable domain comprising or being the amino acid sequence set forth in any one of SEQ ID NOs: 18-27 or an amino acid sequence having at least 80% or at least 90% sequence identity thereto. In some embodiments, the immunoglobulin single variable domain is humanized.

In the present disclosure, "at least 90% (sequence) identity" encompasses at least 90%, at least 91%, at least 92%, at least 93%, at least 94%, at least 95%, at least 96%, at least 97%, at least 98%, at least 99%, or 100% (sequence) identity; "at least 80% (sequence) identity" encompasses at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99%, or 100% (sequence) identity, and a range between any two of the aforementioned values, including integers and decimals.

In some embodiments, the immunoglobulin single variable domain in the aforementioned serum albumin-binding molecule provided by the present disclosure comprises three complementarity-determining regions (CDR1, CDR2, and CDR3) and four FRs, which are arranged from the amino terminus to the carboxyl terminus in the following order: FR1-CDR1-FR2-CDR2-FR3-CDR3-FR4.

In some embodiments, the aforementioned serum albumin-binding molecule provided by the present disclosure is an antibody binding to serum albumin or an antigen-binding fragment thereof, or a conjugate or a fusion protein comprising the antibody or the antigen-binding fragment thereof.

In some embodiments, the aforementioned antibody is a camelid antibody, a chimeric antibody, a humanized antibody, or a fully human antibody.

In some embodiments, the aforementioned antigen-binding fragment is an sdAb or a bispecific antibody, or a multispecific antibody.

In some embodiments, the aforementioned immunoglobulin single variable domain is a VHH.

In some embodiments, the aforementioned immunoglobulin single variable domain is a humanized VHH.

In some embodiments, the aforementioned immunoglobulin single variable domain is an affinity-matured VHH.

In some embodiments, provided is a serum albumin-binding molecule, comprising one or more (e.g. 2, 3, 4, 5, 6, 7, or 8) of the aforementioned immunoglobulin single variable domains, wherein the immunoglobulin single variable domains may be identical or different, and any two of the immunoglobulin single variable domains may be linked directly or by a linker.

In some embodiments, provided is a serum albumin-binding molecule that binds to or competes for binding to the same epitope with the aforementioned immunoglobulin single variable domain of the present disclosure.

In some embodiments, provided is a serum albumin-binding molecule that blocks the binding of the aforementioned immunoglobulin single variable domain of the present disclosure to serum albumin.

In some embodiments, provided is a serum albumin-binding molecule whose binding to serum albumin is blocked by the aforementioned immunoglobulin single variable domain of the present disclosure.

In some embodiments, the aforementioned serum albumin-binding molecule provided by the present disclosure further comprises an Fc region of an immunoglobulin, such as an Fc region selected from the group consisting of human IgG1, IgG2, IgG3, and IgG4; or a histidine tag, such as a (His)₆ tag or a (His)₈ tag.

Fc regions that can be used in the present disclosure may be from immunoglobulins of different subtypes, such as IgG (e.g., subtype IgG1, IgG2, IgG3 or IgG4), IgA1, IgA2, IgD, IgE, or IgM. In some embodiments, the Fc region includes a hinge region or a portion of the hinge region of a constant region, a CH2 region, and a CH3 region.

In some embodiments, the aforementioned immunoglobulin single variable domain may be linked to the Fc region or the histidine tag by a linker. The linker may be a non-functional amino acid sequence with a length of 1-20 or more amino acids without a secondary or higher-order structure. For example, the linker is a flexible linker, such as G₄S (SEQ ID NO: 67), GS, GAP, (G₄S)₂ (SEQ ID NO: 68), (G₄S)₃ (SEQ ID NO: 69), (G₄S)₄ (SEQ ID NO: 70), (G₄S)₅ (SEQ ID NO: 71), ASGS, G₃S, or a combination thereof. In some embodiments, the linker is GGGGSGGGS (SEQ ID NO: 72).

In some embodiments, the serum albumin-binding molecule provided by the present disclosure comprises or is the amino acid sequence set forth in SEQ ID NO: 15 or 16 or an amino acid sequence having at least 80% or at least 90% sequence identity thereto.

In some embodiments, the immunoglobulin single variable domain in the aforementioned serum albumin-binding molecule specifically binds to serum albumin, which may be rodent serum albumin (e.g., mouse serum albumin (mSA)) or primate serum albumin (e.g., cynomolgus monkey serum albumin (cySA) or human serum albumin (HSA)). In some specific embodiments, the serum albumin is HSA.

In some alternatives, the serum albumin-binding molecule of the present disclosure may comprise any one of the aforementioned intact immunoglobulin single variable domains; or a functional portion of any one of the aforementioned immunoglobulin single variable domains, or a variant thereof, such as CDR3, CDR3-FR4, CDR2-FR3-CDR3, CDR2-FR3-CDR3-FR4, FR2-CDR2-FR3-CDR3-FR4, CDR1-FR2-CDR2-FR3-CDR3-FR4, or FR1-CDR1-FR2-CDR2-FR3-CDR3.

In some embodiments, the variant of the functional portion of the immunoglobulin single variable domain may be a polypeptide that retains the serum albumin-binding function of CDR3, CDR3-FR4, CDR2-FR3-CDR3, CDR2-FR3-CDR3-FR4, FR2-CDR2-FR3-CDR3-FR4, CDR1-FR2-CDR2-FR3-CDR3-FR4, or FR1-CDR1-FR2-CDR2-FR3-CDR3 and has at least 80% or at least 90% sequence homology thereto, for example, a polypeptide that retains the serum albumin-binding function of the CDR3 and has a certain degree of sequence homology thereto, such as a polypeptide having at least 80% or at least 90% sequence homology to any one of the CDR3s described above.

In some embodiments, in the serum albumin-binding molecule provided by the present disclosure, the immunoglobulin single variable domain may be used as a carrier for extending the half-life by covalently or non-covalently linking to any other macromolecule or small-molecule compound.

In some embodiments, the serum albumin-binding molecule of the present disclosure comprises one or more therapeutic agents or diagnostic agents, wherein the therapeutic agent or the diagnostic agent is covalently or non-covalently linked to the immunoglobulin single variable domain.

In some embodiments, the therapeutic agent or the diagnostic agent is selected from the group consisting of therapeutic or diagnostic proteins, nucleic acids and small-molecule compounds.

In some embodiments, the serum albumin-binding molecule provided by the present disclosure or the immunoglobulin single variable domain therein binds to serum albumin, such as human serum albumin, with a K_{D} value of ≤ 1 × 10⁻⁷ M, such as ≤ 2 × 10⁻⁸ M, ≤ 1 × 10⁻⁸ M, ≤ 9 × 10⁻⁹ M, ≤ 8 × 10⁻⁹ M, ≤ 7 × 10⁻⁹ M, ≤ 6 × 10⁻⁹ M, ≤ 5 × 10⁻⁹ M, ≤ 4 × 10⁻⁹ M, ≤ 3 × 10⁻⁹ M, ≤ 2 × 10⁻⁹ M, or ≤ 1 × 10⁻⁹ M, or ≤9 × 10⁻¹⁰ M, ≤ 8 × 10⁻¹⁰ M, ≤ 7 × 10⁻¹⁰ M, ≤ 6 × 10⁻¹⁰ M, ≤ 5 × 10⁻¹⁰ M, ≤ 4 × 10⁻¹⁰ M, ≤ 3 × 10⁻¹⁰ M, ≤ 2 × 10⁻¹⁰ M, or ≤ 1 × 10⁻¹⁰ M.

In some embodiments, the serum albumin-binding molecule provided by the present disclosure has an affinity for human serum albumin, with a K_{D} value of 10 nM-100 nM, such as 10 nM-50 nM. Methods for determining the K_{D} values are commonly used in the art, such as the one provided in Example 4 of the present disclosure.

In some embodiments, the serum albumin-binding molecule provided by the present disclosure has an activity selected from at least one of the following:
(a) binding to HSA with a K_{D} value of ≤ 1 × 10⁻⁷ M, such as ≤ 1 × 10⁻⁸ M;
(b) no detectable blocking of the binding of serum albumin to FcRn *in vivo* and/or *in vitro,* wherein the serum albumin is, for example, HSA, and the FcRn is, for example, human FcRn; and
(c) when comprising the therapeutic agent or the diagnostic agent, extending the plasma half-life of the therapeutic agent or the diagnostic agent.

In some embodiments, the serum albumin-binding molecule provided by the present disclosure retains the advantageous properties of a single-domain antibody (e.g., VHH), and has an extended lifespan in the circulation of an individual. Thus, such molecules are capable of circulating in the serum of a subject for several days, thereby reducing the frequency of treatment, the inconvenience to the subject, and the cost of treatment.

In some embodiments, the half-life of the serum albumin-binding molecule of the present disclosure may be controlled by the number of single variable domains present in the molecule that specifically bind to the serum protein.

In some embodiments, the serum albumin-binding molecule of the present disclosure promotes the binding of serum albumin to FcRn *in vivo* and/or *in vitro.*

### Fusion Protein

The present disclosure provides a fusion protein comprising a serum albumin-binding domain and relaxin or an analog thereof, the serum albumin-binding domain comprising at least one immunoglobulin single variable domain, wherein the immunoglobulin single variable domain is any one of the aforementioned immunoglobulin single variable domains of the present disclosure.

In some embodiments, the immunoglobulin single variable domain comprises a CDR1, a CDR2, and a CDR3 as described in any one of the following 1)-5):
1) a CDR1, a CDR2, and a CDR3 from the amino acid sequence set forth in any one of SEQ ID NOs: 6 and 18-27;
2) a CDR1, a CDR2, and a CDR3 from the amino acid sequence set forth in SEQ ID NO: 7;
3) a CDR1, a CDR2, and a CDR3 set forth in SEQ ID NOs: 8, 9, and 10, respectively;
4) a CDR1, a CDR2, and a CDR3 set forth in SEQ ID NOs: 11, 12, and 13, respectively; and
5) a CDR1 of X₁SCX₂G (SEQ ID NO: 64), wherein X₁ is T or N, and X₂ is M or L;
   a CDR2 of X₃IYX₄VGGSTFYX₅X₆SVKG (SEQ ID NO: 58), wherein X₃ is S or T, X₄ is T or M, X₅ is T or A, and X₆ is N or D; and
   a CDR3 of GSPARCX₇X₈RDPTTFX₉Y (SEQ ID NO: 59), wherein X₇ is R or L, X₈ is L or R, and X₉ is D or N;
   wherein one or more of the X₁ to X₉ described above may be a conservative amino acid mutation of an optional amino acid thereof.

The CDRs described above are defined according to the Kabat, IMGT, Chothia, AbM, or Contact numbering scheme, for example, the CDRs are defined according to the Kabat numbering scheme.

In some embodiments, the aforementioned immunoglobulin single variable domain is engineered by any one of the following: camelization or humanization, affinity maturation, T cell epitope removal, reduction of antibody deamidation, reduction of antibody aggregation, and/or reduction of antibody isomerization, or a combination thereof.

In some embodiments, a framework region in the humanized immunoglobulin single variable domain is derived from the human heavy chain variable region germline gene IGHV3-23 or IGVH3-66.

In some specific embodiments, the humanized immunoglobulin single variable domain comprises an amino acid mutation at at least one of the following positions: positions 20, 23, 27, 29, 30, 37, 44, 45, 47, 49, 74, 78, 83, and 84 (positions based on the EU numbering scheme);
for example, the humanized immunoglobulin single variable domain comprises at least one amino acid mutation selected from the group consisting of the following: 20V, 23V, 27N, 29Y, 30L, 37F, 44E, 45R, 47G, 49A, 74A, 78V, 83Q, and 84P;
for example, the humanized immunoglobulin single variable domain comprises a combination of amino acid mutations selected from any one of the following:
   F27N, F29Y, S30L, V37F, G44E, L45R, and W47G;
   L20V, F27N, F29Y, S30L, V37F, G44E, L45R, and W47G;
   A23V, F27N, F29Y, S30L, V37F, G44E, L45R, and W47G;
   F27N, F29Y, S30L, V37F, G44E, L45R, W47G, and S74A;
   F27N, F29Y, S30L, V37F, G44E, L45R, W47G, and L78V;
   F27N, F29Y, S30L, V37F, G44E, L45R, W47G, and S49A;
   F27N, F29Y, S30L, V37F, G44E, L45R, W47G, R83Q, and A84P;
   F27N, F29Y, S30L, V37F, G44E, L45R, W47G, and A84P;
   S30L, V37F, G44E, L45R, W47G, and A84P; or
   S30L, V37F, G44E, L45R, and W47G.

In some embodiments, the immunoglobulin single variable domain comprises or is the amino acid sequence set forth in any one of SEQ ID NOs: 6 and 18-27 or an amino acid sequence having at least 80% or at least 90% sequence identity thereto.

In some embodiments, the immunoglobulin single variable domain comprises or is the amino acid sequence set forth in SEQ ID NO: 7 or an amino acid sequence having at least 80% or at least 90% sequence identity thereto.

In some embodiments, the immunoglobulin single variable domain is a VHH, such as a camelid-derived and humanized VHH.

In some embodiments, the relaxin or the analog thereof in the fusion protein comprises an A chain and a B chain.

In some embodiments, the A chain has the amino acid sequence set forth in any one of SEQ ID NOs: 31, 50, 52, 54, and 56, or an amino acid sequence having at least 90% sequence identity to any one of SEQ ID NOs: 31, 50, 52, 54, and 56, or an amino acid sequence having one or more amino acid additions, deletions, insertions or substitutions compared to any one of SEQ ID NOs: 31, 50, 52, 54, and 56; for example, the A chain may be SEQ ID NO: 31 or 50 with 1, 2, 3, or 4 amino acids deleted from the N-terminus or with 1, 2, 3, or 4 amino acids added to the N-terminus.

In some embodiments, the B chain has the amino acid sequence set forth in any one of SEQ ID NOs: 32, 51, 53, 55, and 57, or an amino acid sequence having at least 90% sequence identity to any one of SEQ ID NOs: 32, 51, 53, 55, and 57, or an amino acid sequence having one or more amino acid additions, deletions, insertions or substitutions compared to any one of SEQ ID NOs: 32, 51, 53, 55, and 57; for example, the B chain may be SEQ ID NO: 51 with 1 amino acid deleted from the N-terminus.

In some embodiments, the A chain and the B chain of the relaxin or the analog thereof are any combination of the above.

In some embodiments, the relaxin or the analog thereof comprises any combination of an A chain and a B chain from any one of the following 1)-7):
1) an A chain set forth in SEQ ID NO: 31 and a B chain set forth in SEQ ID NO: 32;
2) an A chain set forth in SEQ ID NO: 50 and a B chain set forth in SEQ ID NO: 51;
3) an A chain set forth in SEQ ID NO: 52 and a B chain set forth in SEQ ID NO: 53;
4) an A chain set forth in SEQ ID NO: 54 and a B chain set forth in SEQ ID NO: 55;
5) an A chain set forth in SEQ ID NO: 31 and a B chain set forth in SEQ ID NO: 53;
6) an A chain set forth in SEQ ID NO: 52 and a B chain set forth in SEQ ID NO: 32; and
7) an A chain set forth in SEQ ID NO: 56 and a B chain set forth in SEQ ID NO: 57.

> Human RLN2 A chain
   QLYSALANKCCHVGCTKRSLARFC (SEQ ID NO: 31)
> Human RLN2 B chain (delB1)
   SWMEEVIKLCGRELVRAQIAICGMSTWS (SEQ ID NO: 32)
> Human RLN1 A chain
   PYVALFEKCCLIGCTKRSLAKYC (SEQ ID NO: 50)
> Human RLN1 B chain
   VAAKWKDDVIKLCGRELVRAQIAICGMSTWS (SEQ ID NO: 51)
> Human RLN2 A chain (delA1-4)
   ALANKCCHVGCTKRSLARFC (SEQ ID NO: 52)
> Human RLN2 B chain
   DSWMEEVIKLCGRELVRAQIAICGMSTWS (SEQ ID NO: 53)
> Human RLN3 A chain
   DVLAGLSSSCCKWGCSKSEISSLC (SEQ ID NO: 54)
> Human RLN3 B chain
   RAAPYGVRLCGREFIRAVIFTCGGSRW (SEQ ID NO: 55)
> Human RLN2 A chain (Z₁₀LYS)
   X₁₀LYSALANKCCHVGCTKRSLARFC, wherein X₁₀ is Q or D (SEQ ID NO: 56)
> Human RLN2 B chain (E mutated to D)
   DSWMEEVIKLCGRDLVRAQIAICGMSTWS (SEQ ID NO: 57).

In some embodiments, the fusion protein comprises 1 of the aforementioned immunoglobulin single variable domains and 1 of the aforementioned relaxins or analogs thereof. In some embodiments, the fusion protein comprises 1 of the aforementioned immunoglobulin single variable domains and 2 of the aforementioned relaxins or analogs thereof. In some embodiments, the fusion protein comprises 2 of the aforementioned immunoglobulin single variable domains and 1 of the aforementioned relaxins or analogs thereof. In some embodiments, the fusion protein comprises 2 of the aforementioned immunoglobulin single variable domains and 2 of the aforementioned relaxins or analogs thereof.

In some embodiments, the fusion protein comprises a polypeptide shown in any one of or any combination of formula (I) to formula (IV):

B-L₁-A-L₂-VHH formula (I)

A-L₁-B-L₂-VHH formula (II)

VHH-L₂-B-L₁-A formula (III)

VHH-L₁-A-L₁-B formula (IV)

wherein A is any one of the aforementioned relaxin A chains, B is any one of the aforementioned relaxin B chains, and L₁ and L₂ are linkers and may each independently be present or absent.

In some embodiments, L₁ comprises an amino acid sequence selected from the group consisting of (GGGGQ)n (SEQ ID NO: 73), (GGGQ)n, (GGGGS)n, (PGPQ)n, and (PGPA)n, wherein n is selected from the group consisting of integers of 1-10, such as 3, 4, 5, 6, 7, or 8.

In some embodiments, L₁ comprises the amino acid sequence set forth in any one of SEQ ID NOs: 33-35 or an amino acid sequence having at least 90% sequence identity thereto. In some embodiments, L₂ comprises the amino acid sequence of (G_{X}S_{Y})_{Z}, wherein X, Y, and Z are independently selected from the group consisting of integers of 1-10; for example, L₂ is (GGGGS)z, wherein Z is selected from the group consisting of integers of 1-10, such as 3, 4, 5, 6, 7, or 8.

In some embodiments, L₂ comprises the amino acid sequence set forth in SEQ ID NO: 49 or an amino acid sequence having at least 90% sequence identity thereto.

In some embodiments, L₂ comprises an amino acid sequence selected from any one of KR, KRKPTGYGSRKKR (SEQ ID NO: 65), KRKPTGYGSRKR (SEQ ID NO: 66), KRGGGPRR (SEQ ID NO: 60), KRGGGPKR (SEQ ID NO: 61), KRKPTGYGSKR (SEQ ID NO: 62), and KRSLKR (SEQ ID NO: 63), or an amino acid sequence having at least 90% sequence identity thereto.

In some embodiments, the fusion protein is a single-chain protein.

In some embodiments, the fusion protein is a multimeric protein, such as a dimeric protein.

Exemplary embodiments are given below:
A fusion protein, comprising a serum albumin-binding domain and relaxin or an analog thereof, wherein the serum albumin-binding domain comprises an immunoglobulin single variable domain, the immunoglobulin single variable domain comprising a CDR1, a CDR2, and a CDR3 set forth in SEQ ID NOs: 8, 9, and 10, respectively, or a CDR1, a CDR2, and a CDR3 set forth in SEQ ID NOs: 11, 12, and 13, respectively; the relaxin or the analog thereof comprises an A chain and a B chain set forth in SEQ ID NOs: 31 and 32, respectively, or an A chain and a B chain set forth in SEQ ID NOs: 50 and 51, respectively, or an A chain and a B chain set forth in SEQ ID NOs: 52 and 53, respectively, or an A chain and a B chain set forth in SEQ ID NOs: 54 and 55, respectively, or an A chain and a B chain set forth in SEQ ID NOs: 31 and 53, respectively, or an A chain and a B chain set forth in SEQ ID NOs: 52 and 32, respectively, or an A chain and a B chain set forth in SEQ ID NOs: 56 and 57, respectively.

A fusion protein, comprising a serum albumin-binding domain and relaxin or an analog thereof, wherein the serum albumin-binding domain comprises an immunoglobulin single variable domain, the immunoglobulin single variable domain comprising the amino acid sequence set forth in any one of SEQ ID NOs: 6 and 18-27 or an amino acid sequence having at least 90% sequence identity thereto, or the amino acid sequence set forth in SEQ ID NO: 7 or an amino acid sequence having at least 90% sequence identity thereto; the relaxin or the analog thereof comprises an A chain and a B chain set forth in SEQ ID NOs: 31 and 32, respectively, or an A chain and a B chain set forth in SEQ ID NOs: 50 and 51, respectively, or an A chain and a B chain set forth in SEQ ID NOs: 52 and 53, respectively, or an A chain and a B chain set forth in SEQ ID NOs: 54 and 55, respectively, or an A chain and a B chain set forth in SEQ ID NOs: 31 and 53, respectively, or an A chain and a B chain set forth in SEQ ID NOs: 52 and 32, respectively, or an A chain and a B chain set forth in SEQ ID NOs: 56 and 57, respectively.

In some embodiments, provided is a fusion protein, comprising the amino acid sequence set forth in any one of SEQ ID NOs: 36-43 or an amino acid sequence having at least 90% sequence identity thereto.

In some embodiments, the binding affinity of the fusion protein of the present disclosure to the RXFP1 receptor is comparable to that of natural human relaxin-2 (SEQ ID NOs: 31 and 53). In some other embodiments, the binding affinity of the fusion protein of the present disclosure to the RXFP1 receptor is less than that of natural human relaxin-2 (SEQ ID NOs: 31 and 53). In some other embodiments, the binding affinity of the fusion protein of the present disclosure to the RXFP1 receptor is greater than that of natural human relaxin-2 (SEQ ID NOs: 31 and 53).

In some embodiments, the fusion protein of the present disclosure shows significant improvements compared to natural human relaxin-2 (SEQ ID NOs: 31 and 53); for example, the fusion protein has an *in vivo* half-life (t_{1/2}) comparable to that of *in vivo* serum albumin, ranging from a dozen hours to a dozen days across different species.

In some embodiments, the fusion protein of the present disclosure binds to HSA with a K_{D} value of ≤ 1 × 10⁻⁷ M, such as ≤ 1 × 10⁻⁸ M.

In some embodiments, the fusion protein of the present disclosure does not block the binding of serum albumin to FcRn *in vivo* and/or *in vitro.*

The fusion protein of the present disclosure encompasses pharmaceutically acceptable salts thereof.

### Polynucleotide and Vector

In some embodiments, provided is a polynucleotide encoding the serum albumin-binding molecule of the present disclosure.

In some embodiments, provided is a polynucleotide encoding the fusion protein of the present disclosure.

The polynucleotide may be an RNA, a DNA, or a cDNA. According to some embodiments of the present disclosure, the polynucleotide of the present disclosure is an isolated polynucleotide.

The polynucleotide of the present disclosure may be in the form of, may be present in, and/or may be part of a vector, such as a plasmid, cosmid, YAC, or viral vector. The vector may especially be an expression vector, i.e., a vector that provides for the *in vitro* and/or *in vivo* (i.e., in suitable host cells, host organisms and/or expression systems) expression of the serum albumin-binding molecule. The expression vector generally comprises at least one of the polynucleotides of the present disclosure, which is operably linked to one or more suitable expression regulatory elements (e.g., promoters, enhancers, terminators, and the like). The selection of the elements and their sequences for expression in a particular host is within the knowledge of those skilled in the art. Regulatory elements and other elements useful or necessary for the expression of the serum albumin-binding molecule of the present disclosure include, for example, promoters, enhancers, terminators, integration factors, selection labels, leader sequences, and reporter genes.

The polynucleotide of the present disclosure may be prepared or obtained by known means (e.g., by automatic DNA synthesis and/or recombinant DNA techniques) based on information on the amino acid sequence of the polypeptide of the present disclosure, and/or may be isolated from a suitable natural source.

### Host Cell

In some embodiments, provided are a host cell expressing or capable of expressing one or more of the serum albumin-binding molecules of the present disclosure, and/or a recombinant host cell comprising the polynucleotide or the vector of the present disclosure.

In some embodiments, the host cell is a bacterial cell, a fungal cell, or a mammalian cell. Bacterial cells include, for example, cells of gram-negative bacterial strains (e.g., *Escherichia coli* strains, *Proteus* strains, and *Pseudomonas* strains), and gram-positive bacterial strains (e.g., *Bacillus* strains, *Streptomyces* strains, *Staphylococcus* strains, and *Lactococcus* strains).

Fungal cells include, for example, cells of species of *Trichoderma, Neurospora,* and *Aspergillus;* or cells of species of *Saccharomyces* (e.g., *Saccharomyces cerevisiae*), *Schizosaccharomyces* (e.g., *Schizosaccharomyces pombe*), *Pichia* (*Pichia pastoris* and *Pichia methanolica*), and *Hansenula.*

Illustratively, mammalian cells include, for example, HEK293 cells, CHO cells, BHK cells, HeLa cells, COS cells, and the like.

Amphibian cells, insect cells, plant cells, and any other cells used in the art for expressing heterologous proteins may also be used in the present disclosure.

The cells of the present disclosure are unable to develop into complete plants or animal individuals.

### Production or Preparation Method

The present disclosure provides a method for preparing the serum albumin-binding molecule or the fusion protein of the present disclosure, comprising:
- culturing the host cell of the present disclosure under conditions that allow expression of the serum albumin-binding molecule or the fusion protein of the present disclosure; and
- collecting the serum albumin-binding molecule or the fusion protein expressed by the host cell from the culture; and
- optionally, further purifying and/or modifying the serum albumin-binding molecule or the fusion protein of the present disclosure.

The serum albumin-binding molecule or the fusion protein of the present disclosure can be produced intracellularly (e.g., in the cytoplasm, in the periplasm, or in inclusion bodies) in the cell as described above, followed by isolation from the host cell and optionally further purification; or it may be produced extracellularly (e.g., in the medium in which the host cell is cultured), followed by isolation from the medium and optionally further purification.

Methods and reagents for recombinant production of polypeptides or proteins, such as specific suitable expression vectors, transformation or transfection methods, selection labels, methods for inducing protein expression, culture conditions, and the like, are known in the art. Similarly, isolation and purification techniques suitable for use in the manufacture of proteins of interest, such as the binding molecules or antibodies of the present disclosure, are well known to those skilled in the art. Methods for producing and purifying antibodies are well known in the prior art and can be found in, for example, Antibodies: A Laboratory Manual, Cold Spring Harbor Press (chapters 5-8 and 15). The engineered antibodies of the present disclosure may also be prepared and purified by conventional methods. For example, cDNA sequences encoding the heavy and light chains can be cloned and recombined into an expression vector. Recombinant immunoglobulin expression vectors can be used to stably transfect cells. Mammalian expression systems may result in glycosylation of antibodies, particularly at the highly conserved N-terminus of the Fc region. Stable clones are obtained by expression of antibodies specifically binding to human antigens. Positive clones are expanded in a serum-free culture medium in a bioreactor to produce antibodies. The culture medium with the secreted antibodies can be purified and collected by conventional techniques. The antibodies can be filtered and concentrated by conventional methods. Soluble mixtures and multimers can also be removed by conventional methods, such as molecular sieves and ion exchange. The resulting products need to be immediately frozen, such as at -70 °C, or lyophilized.

However, the serum albumin-binding molecule or the fusion protein of the present disclosure may also be obtained by other methods for producing proteins known in the art, such as chemical synthesis, including solid-phase or liquid-phase synthesis.

### Composition

In some embodiments, the composition is a pharmaceutical composition comprising a prophylactically or therapeutically effective amount of the serum albumin-binding molecule or the fusion protein of the present disclosure, and/or the polynucleotide encoding the serum albumin-binding molecule or the fusion protein as described above, and one or more pharmaceutically acceptable carriers, diluents, buffers or excipients.

In some specific embodiments, a unit dose of the pharmaceutical composition may comprise 0.01 to 99 wt% of the serum albumin-binding molecule or the fusion protein. In some other specific embodiments, the unit dose of the pharmaceutical composition comprises the serum albumin-binding molecule or the fusion protein in an amount of 0.1-2000 mg, and in some embodiments, 1-1000 mg.

### Kit and Detection

The present disclosure provides a kit or product comprising any of the aforementioned serum albumin-binding molecules of the present disclosure and/or the nucleic acid molecules encoding the serum albumin-binding molecules of the present disclosure.

The present disclosure provides a composition for detecting serum albumin, the composition comprising the serum albumin-binding molecule of the present disclosure. The present disclosure further provides a method, system or device for detecting serum albumin *in vivo* or *in vitro,* comprising the use of the serum albumin-binding molecule of the present disclosure.

In some embodiments, the *in vitro* detection method, system or device may, for example, comprise (1) contacting a sample with the serum albumin-binding molecule of the present disclosure; (2) detecting formation of a complex between the serum albumin-binding molecule of the present disclosure and the sample; and/or (3) contacting a reference sample (e.g., a control sample) with an antibody; and (4) determining the extent of complex formation between the antibody and the sample by comparison with the reference sample. A change (e.g., a statistically significant change) in complex formation in a sample or subject as compared to a control sample or subject indicates the presence of serum albumin in the sample.

In some other embodiments, the *in vivo* detection method, system or device may comprise: (1) administering to a subject the serum albumin-binding molecule of the present disclosure; and (2) detecting formation of a complex between the serum albumin-binding molecule of the present disclosure and the subject. The detection may include determining the location or time at which the complex is formed. The antibody binding to the serum albumin-binding molecule may be directly or indirectly labeled with a detectable substance to facilitate detection of bound or unbound antibody. Suitable detectable substances include various enzymes, prosthetic groups, fluorescent substances, luminescent substances, and radioactive substances. The complex formation between the serum albumin-binding molecule of the present disclosure and serum albumin can be detected by determining or visualizing the antibody that is bound or unbound to the serum albumin-binding molecule. Conventional detection assays may be used, such as enzyme-linked immunosorbent assay (ELISA), radioimmunoassay (RIA), or tissue immunohistochemistry. In some embodiments, the sample is analyzed for the presence of serum albumin by a competitive immunoassay that uses a standard labeled with a detectable substance and an unlabeled serum albumin-binding molecule of the present disclosure. The living sample to be detected or assayed may be histiocyte, blood, plasma, serum, pancreatic juice, urine, feces, tissue fluid, or culture fluid.

In some embodiments, the serum albumin-binding molecule of the present disclosure may be labeled with a fluorophore or a chromophore for detection purposes.

### Method for Treating and Preventing Disease and Pharmaceutical Use

The present disclosure provides a method for extending the plasma half-life of a therapeutic agent or a diagnostic agent, comprising covalently or non-covalently linking the serum albumin-binding molecule or the immunoglobulin single variable domain thereof of the present disclosure to the therapeutic agent or the diagnostic agent. Methods for their linkage are known in the art or may be any future method, such as fusion by chemical coupling, fusion at the DNA level, fusion at the mRNA level, fusion at the protein level, or the like.

In some embodiments, the therapeutic or diagnostic molecule may be a therapeutic target or a diagnostic target. The target may be any protein, peptide, nucleic acid, oligonucleotide, saccharide, polysaccharide or glycoprotein for therapeutic or diagnostic use. Examples include, but are not limited to, receptors, receptor ligands, viral coat proteins, immune system proteins, hormones, enzymes, antigens, cell signaling proteins, or fragments thereof.

In some embodiments, the plasma half-life of the therapeutic agent or the diagnostic agent linked to the serum albumin-binding molecule of the present disclosure or the immunoglobulin single variable domain thereof is at least 1.5 times greater, preferably at least 2 times greater, for example, at least 5 times greater, such as at least 10 times or more than 20 times greater, as compared to before the linkage; for example, the increased plasma half-life may be greater than 1 h, preferably greater than 2 h, more preferably greater than 6 h, such as greater than 12 h, or even greater than 24 h, 48 h or 72 h, as compared to before the linkage.

The present disclosure provides a method for treating and/or preventing a disease, comprising administering to a subject in need thereof a therapeutically and/or prophylactically effective amount of the serum albumin-binding molecule or the fusion protein of the present disclosure.

The present disclosure further provides a method for treating and/or preventing a disease by using a serum albumin-binding molecule or a fusion protein, comprising administering to a subject in need thereof a therapeutically and/or prophylactically effective amount of the serum albumin-binding molecule or the fusion protein of the present disclosure.

The present disclosure further provides use of the serum albumin-binding molecule in the preparation of a medicament for treating and/or preventing a disease.

In some embodiments, the disease is a fibrotic disease or a cardiovascular disease.

In some specific embodiments, the cardiovascular disease is heart failure or myocardial hypertrophy.

### BRIEF DESCRIPTION OF THE DRAWINGS

FIGs. 1A to 1C show the ELISA binding assay results for serum albumin-binding molecules.
FIG. 1A shows the ELISA binding assay results for the binding of anti-HSA antibodies to human serum albumin (HSA).
FIG. 1B shows the ELISA binding assay results for the binding of anti-HSA antibodies to monkey serum albumin (CySA).
FIG. 1C shows the ELISA binding assay results for the binding of anti-HSA antibodies to mouse serum albumin (mSA).
FIGs. 2A and 2B show the assay results for the effects of anti-HSA antibodies on the binding of FcRn to HSA.
FIG. 3 shows the plasma concentration-time curves following intravenous administration in a pharmacokinetic study for anti-HSA antibodies in mice.
FIGs. 4A to 4B show the results of a pharmacokinetic study for anti-HSA antibodies in mice.

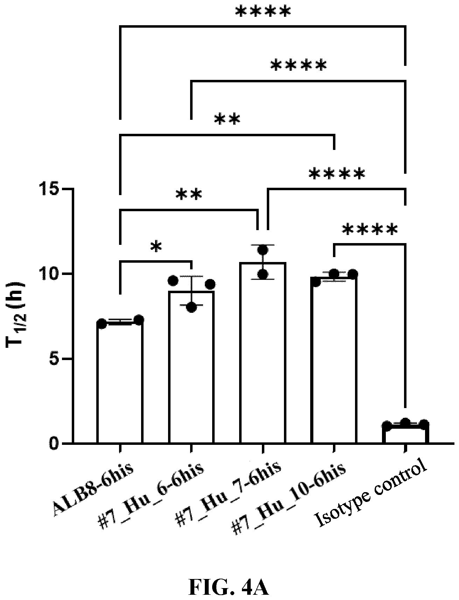

FIG. 4A shows the comparison results for T_{1/2} parameters of anti-HSA antibodies in plasma calculated using a non-compartmental model.

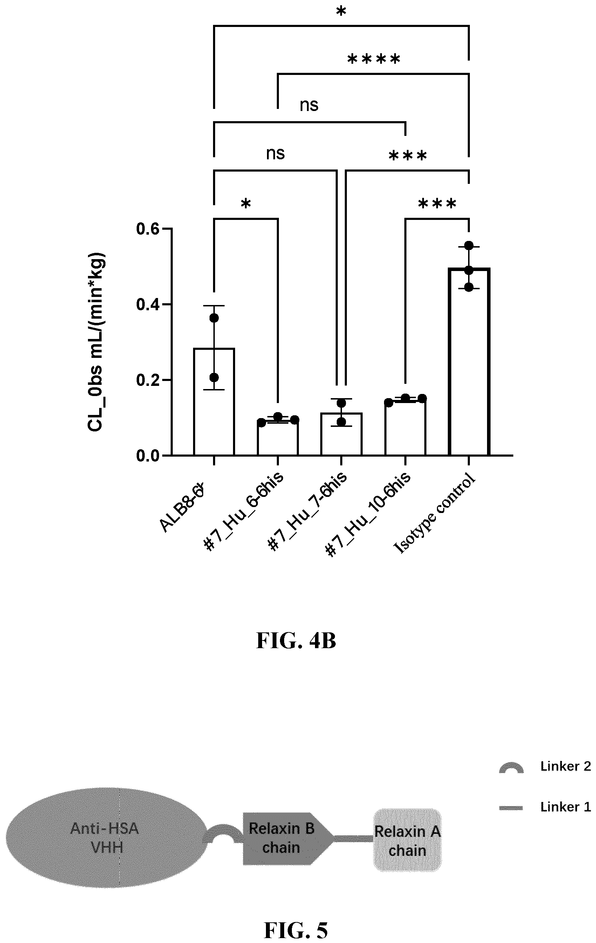

FIG. 4B shows the comparison results for Cl_obs (mL/(min*kg)) parameters.
FIG. 5 shows a structural schematic diagram of the fusion protein (formula (III)) of the present disclosure.

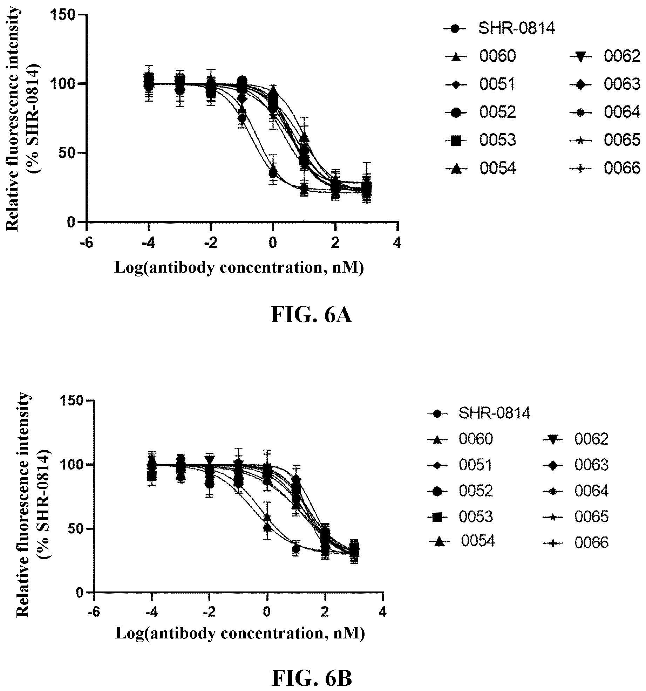

FIG. 6A shows the detection results for the *in vitro* activity of fusion proteins in THP1 cells in the absence of HSA.
FIG. 6B shows the detection results for the *in vitro* activity of fusion proteins in THP1 cells in a system containing 1% HSA.

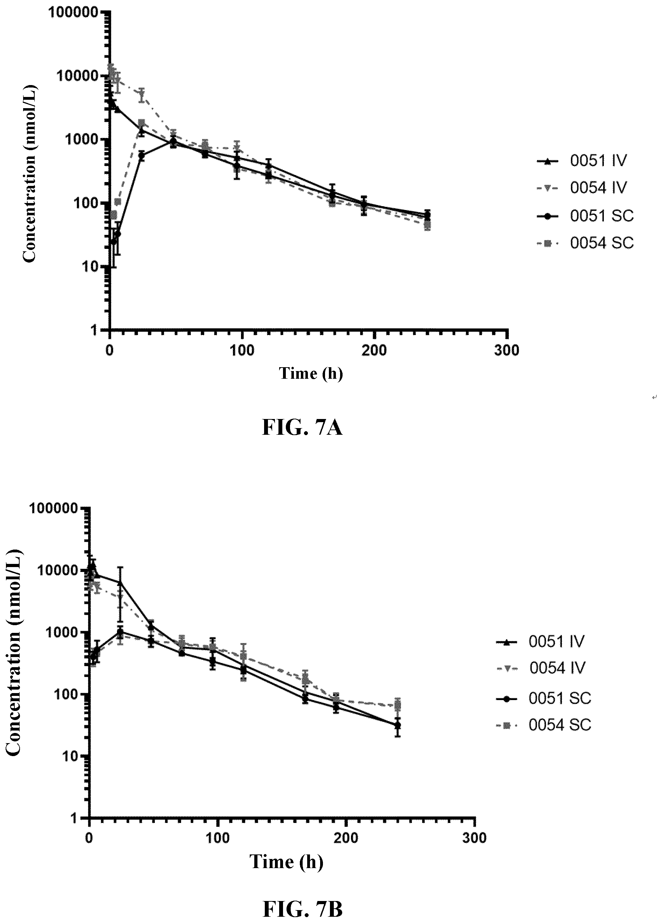

FIG. 7A shows the plasma concentration-time curves of fusion proteins in a mouse model.
FIG. 7B shows the plasma concentration-time curves of fusion proteins in a rat model.

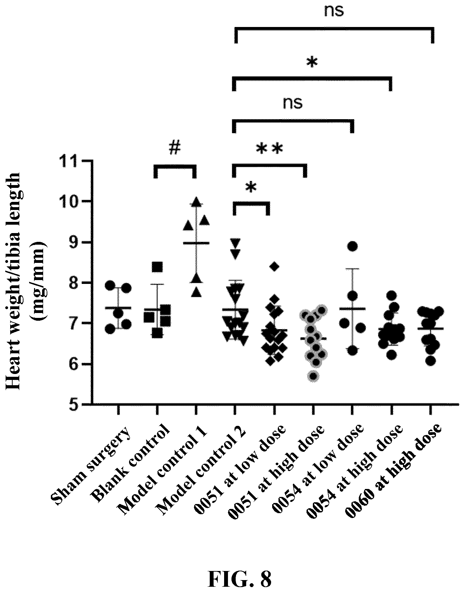

FIG. 8 shows the heart weight/tibia length ratios of a mouse ISO myocardial hypertrophy model in an experiment on the effects of fusion proteins on myocardial hypertrophy in mice.

### DETAILED DESCRIPTION

### Definition

In order to facilitate the understanding of the present disclosure, some technical and scientific terms are specifically defined below. Unless otherwise specifically defined herein, all other technical and scientific terms used herein have the meanings generally understood by those of ordinary skill in the art to which the present disclosure belongs. The three-letter and single-letter codes for amino acids used herein are as described in J. biol. chem., 243, p3558 (1968).

"Antibody" or "immunoglobulin", whether referring to a heavy-chain antibody or to a conventional four-peptide-chain antibody formed by two heavy chains and two light chains linked by interchain disulfide bonds, is used as a general term to include full-length antibodies, individual chains thereof, and all portions, domains or fragments thereof (including, but not limited to, antigen-binding domains or fragments, such as VHH domains or VH/VL domains).

"Sequence" (e.g., in terms such as "immunoglobulin sequence", "antibody sequence", "single variable domain sequence", "VHH sequence", or "protein sequence") is generally intended to include both related amino acid sequences and nucleic acid or nucleotide sequences encoding the sequences, unless a further limited interpretation is required in the present disclosure.

"Domain" of a polypeptide or protein refers to a folded protein structure that is capable of maintaining its tertiary structure independently of the rest of the protein. In general, a domain is responsible for a single functional property of a protein, and in many cases may be added, deleted, or transferred to other proteins without loss of functions of the rest of the protein and/or the domain.

"Immunoglobulin domain" refers to a globular region of an antibody chain.

"Immunoglobulin variable domain" refers to a domain consisting essentially of "framework region 1" (FR1), "framework region 2" (FR2), "framework region 3" (FR3), and "framework region 4" (FR4), as well as "complementarity-determining region 1" (CDR1), "complementarity-determining region 2" (CDR2), and "complementarity-determining region 3" (CDR3). Thus, the general structure (or sequence) of a variable domain can be expressed as follows: FR1-CDR1-FR2-CDR2-FR3-CDR3-FR4. A variable domain possesses specificity for an antigen by virtue of having an antigen-binding site.

"Antibody framework (FR)" refers to a portion of a variable domain, which serves as a framework for the antigen-binding loops (CDRs) of the variable domain.

"Immunoglobulin single variable domain" is generally used to refer to an immunoglobulin variable domain (which may be a heavy or light chain domain, including a VH, VHH, or VL domain) that can form a functional antigen-binding site without interacting with other variable domains (e.g., without VH/VL interactions as are required between the VH and VL domains of conventional four-chain monoclonal antibodies).

Examples of "immunoglobulin single variable domains" include nanobodies (including VHHs, humanized VHHs, and/or camelized VHs, e.g., camelized human VHs), IgNARs, domains, (single-domain) antibodies as VH domains or derived from VH domains (such as dAbs^{™}) and (single-domain) antibodies as VL domains or derived from VL domains (such as dAbs^{™}). Immunoglobulin single variable domains based on and/or derived from heavy chain variable domains (such as VH or VHH domains) are generally preferred. A specific example of an immunoglobulin single variable domain is a "VHH domain" (or simply "VHH") as defined below.

"VHH domain", also known as heavy chain single-domain antibody, VHH, V_{HH} domain, VHH antibody fragment, VHH antibody, or nanobody, is a variable domain of an antigen-binding immunoglobulin known as a "heavy-chain antibody" (i.e., "an antibody devoid of light chains") (Hamers-Casterman C, Atarhouch T, Muyldermans S, Robinson G, Hamers C, Songa EB, Bendahman N, Hamers R., "Naturally occurring antibodies devoid of light chains"; Nature 363, 446-448 (1993)). The term "VHH domain" is used to distinguish said variable domain from the VHs and VLs present in antibodies of the conventional four-peptide-chain structure. VHH domains specifically bind to epitopes without the need for additional antigen-binding domains (for the VH or VL domains in antibodies of the conventional four-peptide-chain structure, epitopes are recognized by the VL domains together with the VH domains). A VHH domain is a small, stable, and efficient antigen recognition unit formed by a single immunoglobulin domain. The terms "heavy-chain single-domain antibody", "VHH domain", "VHH", "VHH domain", "VHH antibody fragment", "VHH antibody", "Nanobody^{®}" and "Nanobody^{®} domain" ("Nanobody" is a trademark of Ablynx N.V., Ghent, Belgium) are used interchangeably. "VHH domains" include, but are not limited to, natural antibodies produced by camelids, antibodies produced by camelids and then humanized, or antibodies obtained by screening with phage display techniques. The total number of amino acid residues in a VHH domain will usually be in the range of 110 to 120, often between 112 and 115. However, it should be noted that smaller and longer sequences may also be suitable for the purposes described in the present disclosure. Methods for obtaining VHHs that bind to a particular antigen or epitope have been previously disclosed in the following documents: R. van der Linden et al., Journal of Immunological Methods, 240(2000) 185-195; Li et al., J Biol Chem., 287(2012) 13713-13721; Deffar et al., African Journal of Biotechnology Vol. 8(12), pp. 2645-2652, 17 June, 2009 and WO94/04678.

As is well known in the art for VH domains and VHH domains, the total number of amino acid residues in each of the CDRs may vary and may not correspond to the total number of amino acid residues indicated by the Kabat numbering (that is, one or more positions according to the Kabat numbering may not be occupied in the actual sequence, or the actual sequence may contain more amino acid residues than the number allowed for by the Kabat numbering). This means that, in general, the numbering according to Kabat may or may not correspond to the actual numbering of the amino acid residues in the actual sequence. Other numbering systems or numbering schemes include Chothia, IMGT, and AbM.

"Humanized antibody", also known as a CDR-grafted antibody, refers to an antibody produced by grafting non-human CDR sequences into the framework of variable regions of a human antibody. Chimeric antibodies, due to their significant non-human protein content, can induce a strong immune response. This issue can be overcome by using humanized antibodies. To avoid the decrease in activity caused by the decrease in immunogenicity, the variable regions of a fully human antibody can be subjected to minimum reverse mutation to maintain activity. Examples of "humanization" include "humanization" of a VHH domain derived from camelidae by replacing one or more amino acid residues in the amino acid sequence of the original VHH sequence with one or more amino acid residues present at the corresponding positions in a VH domain of a human antibody of the conventional four-peptide-chain structure; the humanized VHH domain may contain one or more fully human framework region sequences, and in some specific embodiments, may contain the human framework region sequence of IGHV3. Methods for humanization include, for example, protein surface amino acid humanization (resurfacing) and universal framework grafting method for antibody humanization (CDR grafting to a universal framework), i.e., "grafting" CDRs onto other "frameworks" (including but not limited to human scaffolds or non-immunoglobulin scaffolds). Scaffolds and techniques suitable for such CDR grafting are known in the art. For example, germline DNA sequences of genes of the human heavy and light chain variable regions can be found in the VBase human species sequence database, as well as in Kabat, E. A. et al., 1991 Sequences of Proteins of Immunological Interest, 5th edition. The humanized antibody of the present disclosure also includes humanized antibodies formed by further affinity maturation of the CDRs by phage display. Additionally, in order to avoid the decrease in activity caused by the decrease in immunogenicity, the framework region sequence in the human antibody variable region can be subjected to minimum reverse mutation or back mutation to maintain activity.

Those skilled in the art can determine the amino acid sequence boundaries of antibody CDRs using any one of many known numbering schemes, including the numbering schemes described in the following documents: Kabat et al., supra (the "Kabat" numbering scheme); Al-Lazikani et al., 1997, J. Mol. Biol., 273:927-948 (the "Chothia" numbering scheme); MacCallum et al., 1996, J. Mol. Biol., 262:732-745 (the "Contact" numbering scheme); Lefran et al., Dev. Comp. Immunol., 2003, 27:55-77 (the "IMGT" numbering scheme), and Honegge and Plückthun, J. Mol. Biol., 2001, 309:657-70 (the "AHo" numbering scheme), each of which is incorporated herein by reference in its entirety.

"Epitope" or the term "antigenic determinant" used interchangeably herein refers to any antigenic determinant on an antigen to which an antibody binds. An antigenic determinant generally comprises chemically active surface groups of molecules, such as amino acids or sugar side chains, and usually has specific three-dimensional structural characteristics and/or specific charge characteristics. For example, an epitope typically comprises at least 3, 4, 5, 6, 7, 8, 9, 10, 11, 12, 13, 14, or 15 contiguous or non-contiguous amino acids in a unique spatial conformation, and it may be a "linear" epitope or a "conformational" epitope. See, e.g., Epitope Mapping Protocols in Methods in Molecular Biology, volume 66, G.E. Morris, Ed. (1996). In a linear epitope, all points of interaction between an antigen and an interacting molecule (e.g., an antibody) exist linearly along the primary amino acid sequence of the antigen. In a conformational epitope, the points of interaction exist across amino acid residues that are separated from one another. Epitopes of a given antigen can be identified using many epitope mapping techniques well known in the art. See, e.g., Epitope Mapping Protocols in Methods in Molecular Biology, volume 66, G.E. Morris, Ed. (1996). By way of example, linear epitopes can be determined, for example, by the following method: simultaneously synthesizing on a solid support a large number of peptides corresponding to portions of a protein molecule, and allowing these peptides to react with an antibody while still attached to the support. Such techniques are known in the art and are described, for example, in U.S. Patent No. 4,708,871; Geysen et al. (1984) Proc. Natl. Acad. Sci. USA 81:3998-4002; and Geysen et al. (1986) Molec. Immunol. 23:709-715. Conformational epitopes can also be identified, for example, by determining the spatial configuration of amino acids using methods such as X-ray crystallography and two-dimensional nuclear magnetic resonance. Antibodies can be competitively screened for binding to the same epitope using conventional techniques known to those skilled in the art. For example, competition and cross-competition studies can be performed to obtain antibodies that compete or cross-compete with one another for binding to an antigen. A high-throughput method for obtaining antibodies that bind to the same epitope based on their cross-competition is described in International Patent Application No. WO03/48731. Thus, an antibody and an antigen-binding fragment thereof that compete for binding to the same epitope on serum albumin with the antibody molecule of the present disclosure can be obtained by conventional techniques known to those skilled in the art.

In general, the term "specificity" refers to the number of different types of antigens or epitopes to which a particular antigen-binding molecule or antigen-binding protein (e.g., the serum albumin-binding molecule of the present disclosure) can bind. The specificity of an antigen-binding protein can be determined based on its affinity and/or avidity. Affinity, expressed as the dissociation equilibrium constant (K_{D}) between an antigen and an antigen-binding protein, is a measure of the binding strength between an epitope and an antigen-binding site on the antigen-binding protein: the smaller the K_{D} value, the stronger the binding strength between the epitope and the antigen-binding protein (or affinity can also be expressed as the association constant (Kₐ), which is 1/K_{D}). As will be appreciated by those skilled in the art, affinity can be determined in a known manner depending on the specific antigen of interest. Avidity is a measure of the binding strength between an antigen-binding protein (e.g., an immunoglobulin, an antibody, an immunoglobulin single variable domain, or a polypeptide comprising same) and a related antigen. Avidity is related to both the affinity for its antigen-binding site on the antigen-binding protein and the number of related binding sites present on the antigen-binding protein.

"Serum albumin-binding domain" means any polypeptide capable of specifically binding to serum albumin or an epitope thereof. "Serum albumin-binding molecule" means any molecule, including but not limited to a protein and a polypeptide, that is capable of specifically binding to serum albumin or an epitope thereof. The serum albumin-binding molecule may include an antibody or an antigen-binding fragment thereof, as defined in the present disclosure, that is directed against serum albumin or an epitope thereof, or a conjugate or fusion protein comprising the antibody or the antigen-binding fragment thereof. The antigen-binding fragment is, for example, an sdAb or a bispecific antibody, or a multispecific antibody. The serum albumin-binding molecule of the present disclosure may comprise at least one (e.g., 2, 3, 4, or more) immunoglobulin single variable domain (e.g., VHH) that binds to serum albumin. In addition to the immunoglobulin single variable domain, the serum albumin-binding molecule of the present disclosure may also comprise a linker and/or a portion having the function of an effector molecule. The effector molecule includes, but is not limited to, a diagnostic agent or a therapeutic agent, such as an anti-tumor agent, an immunomodulator, a chromophore, a fluorophore, a chemiluminescent compound, an enzyme, a metal ion, and any combination thereof.

An "affinity-matured" serum albumin antibody (e.g., VHH) has one or more changes in one or more CDRs, which result in an increase in the affinity for serum albumin as compared to its parent anti-serum albumin antibody. Affinity-matured anti-serum albumin antibodies can be prepared, for example, by methods known in the art as described below: Marks et al., 1992, Biotechnology 10:779-783 or Barbas et al., 1994, Proc. Nat. Acad. Sci, USA 91:3809-3813; Shier et al., 1995, Gene 169:147-155; Yelton et al., 1995, Immunol. 155:1994-2004; Jackson et al., 1995, J. Immunol. 154(7):3310-9; Hawkins et al., 1992, J. MoI. Biol. 226(3):889896; and KS Johnson and RE Hawkins, "Affinity maturation of antibodies using phage display", Oxford University Press 1996.

"Back mutation" refers to the mutation of an FR amino acid residue derived from a human antibody to an amino acid residue at a corresponding position of the original source antibody, which is usually performed to prevent a decrease in activity while the humanization of the antibody causes a decrease in immunogenicity. The variable region of the humanized antibody may be subjected to minimum reverse mutation to maintain the activity of the antibody.

Generally, the serum albumin-binding molecule of the present disclosure will bind to the antigen to be bound (i.e., serum albumin) with a dissociation constant (K_{D}) of preferably 10⁻⁷ to 10⁻¹⁰ mol/L (M), more preferably 10⁻⁸ to 10⁻¹⁰ mol/L, and even more preferably 10⁻⁹ to 10⁻¹⁰ or lower, and/or with an association constant (Kₐ) of at least 10⁻⁷ M, preferably at least 10⁻⁸ M, more preferably at least 10⁻⁹ M, and more preferably at least 10⁻¹⁰ M, as measured in a Biacore, KinExA or Fortibio assay. Any K_{D} value greater than 10⁻⁴ M is generally considered to indicate non-specific binding. Specific binding of an antigen-binding protein to an antigen or epitope can be measured in any suitable manner known, including, for example, surface plasmon resonance (SPR) assay, Scatchard assay, and/or competitive binding assay (e.g., radioimmunoassay (RIA), enzyme immunoassay (ELISA), and sandwich competitive assay) described in the present disclosure.

The terms "inhibition" and "blocking" are used interchangeably and encompass both partial and complete inhibition/blocking.

In the present disclosure, "homology" or "identity" refers to sequence similarity between two polynucleotide sequences or between two polypeptides. When positions in two compared sequences are occupied by identical bases or amino acid monomer subunits, for example, if the position of each of two DNA molecules is occupied by adenine, the molecules are homologous at that position. The homology percentage between two sequences is a function of the number of matching or homologous positions shared by the two sequences divided by the number of positions compared × 100%. For example, in the optimal alignment of sequences, if 6 out of 10 positions of two sequences are matched or homologous, the two sequences are 60% homologous, and if 95 out of 100 positions of two sequences are matched or homologous, the two sequences are 95% homologous. In general, when two sequences are aligned, comparison is performed to obtain the maximum homology percentage.

"Conservative amino acid mutation" or "conservative (amino acid) substitution" refers to the conservative amino acid replacement of one or more amino acid residues in a protein or polypeptide, where the amino acid residue before the replacement is similar in chemical structure to the amino acid residue after the replacement, and the replacement has little or substantially no effect on the function, activity or other biological properties of the protein or polypeptide. The conservative amino acid replacement is well known in the art; for example, a conservative amino acid substitution is preferably substitution of one amino acid from the following groups (i)-(v) with another amino acid residue from the same group:
(i) relatively small, aliphatic, non-polar or weakly polar residues: Ala, Ser, Thr, Pro, and Gly;
(ii) polar, negatively charged residues and (uncharged) amides thereof: Asp, Asn, Glu, and Gln;
(iii) polar, positively charged residues: His, Arg, and Lys; (iv) relatively large, aliphatic, nonpolar residues: Met, Leu, Ile, Val, and Cys; and
(v) aromatic residues: Phe, Tyr, and Trp.

Particularly preferably, the conservative amino acid substitution is as follows: substitution of Ala with Gly or Ser; substitution of Arg with Lys; substitution of Asn with Gln or His; substitution of Asp with Glu; substitution of Cys with Ser; substitution of Gln with Asn; substitution of Glu with Asp; substitution of Gly with Ala or Pro; substitution of His with Asn or Gln; substitution of Ile with Leu or Val; substitution of Leu with Ile or Val; substitution of Lys with Arg, Gln, or Glu; substitution of Met with Leu, Tyr, or Ile; substitution of Phe with Met, Leu, or Tyr; substitution of Ser with Thr; substitution of Thr with Ser; substitution of Trp with Tyr; substitution of Tyr with Trp or Phe; or substitution of Val with Ile or Leu.

"Nucleic acid", "nucleic acid molecule", and "polynucleotide" are used interchangeably herein to refer to any DNA or RNA molecule, either single- or double-stranded, and, if single-stranded, the molecule of its complementary sequence, preferably a double-stranded DNA. A nucleic acid is "operably linked" when it is placed into a functional relationship with another nucleic acid sequence. For example, a promoter or enhancer is operably linked to a coding sequence if it affects the transcription of the coding sequence. The term "vector" refers to a nucleic acid molecule capable of transporting another nucleic acid to which it has been linked. In one embodiment, the vector is a "plasmid" that refers to a circular double-stranded DNA loop into which additional DNA segments may be ligated. In another embodiment, the vector is a viral vector, in which additional DNA segments may be ligated into the viral genome. The vectors in the present disclosure are capable of autonomously replicating in a host cell into which they have been introduced (e.g., bacterial vectors having a bacterial origin of replication and episomal mammalian vectors) or can be integrated into the genome of a host cell upon introduction into the host cell and thus replicated along with the host genome (e.g., non-episomal mammalian vectors).

The expressions "cell", "cell line", and "cell culture" used herein are used interchangeably, and all such designations include their progeny. Thus, "transformant" and "transformed cell" include primary test cells and cultures derived therefrom, regardless of the number of passages. It should also be understood that all progeny may not be precisely identical in DNA content due to intentional or unintentional mutations. Mutant progeny with identical function or biological activity as those screened in the initially transformed cells are included. When referring to different designations, they will become clear from the context.

"Host cell" includes individual cells or cell cultures, which may be or have been the recipient of a vector for incorporation of a nucleic acid insert. The host cell includes progeny of a single host cell, and the progeny may not necessarily be identical (in morphology or genomic DNA complement) to the original parent cell due to natural, accidental or intentional mutations. The host cell includes cells transfected and/or transformed *in vivo* with the nucleic acid of the present disclosure. "Cell", "cell line", and "cell culture" are used interchangeably, and all such designations include their progeny. It should also be understood that all progeny may not be precisely identical in DNA content due to intentional or unintentional mutations. Mutant progeny with identical function or biological activity as those screened in the initially transformed cells are included.

"Pharmaceutical composition" refers to a mixture containing one or more of the fusion proteins, the serum albumin-binding molecules, and the polynucleotides described in the present disclosure, together with other components, such as physiologically/pharmaceutically acceptable carriers, diluents, buffers or excipients. The pharmaceutical composition is intended to promote administration to an organism and facilitate the absorption of the active ingredient so that it can exert its bioactivity.

"Giving", "administering", and "treating", when applied to animals, humans, subjects, cells, tissues, organs, or biological fluids, refer to contact of an exogenous drug, a therapeutic agent, a diagnostic agent, or a composition with the animals, humans, subjects, cells, tissues, organs or biological fluids. "Giving", "administering", and "treating" can refer to, for example, therapeutic, pharmacokinetic, diagnostic, research, and experimental methods. The treatment of cells comprises contacting a reagent with cells and contacting the reagent with a fluid, where the fluid is in contact with the cells. "Giving", "administering", and "treating" also mean treating a cell, by a reagent, diagnosis, a binding composition, or by another cell *in vitro* and *ex vivo.* "Treating", when applied to humans, veterinary or research subjects, refers to therapeutic treatment, preventive or prophylactic measures, and research and diagnostic applications.

"Treatment" means administering a therapeutic agent (e.g., the fusion protein of the present disclosure) either internally or externally to a subject having one or more disease symptoms on which the therapeutic agent is known to have a therapeutic effect. Generally, the therapeutic agent is administered in an amount effective to alleviate one or more disease symptoms in the patient or population being treated to induce regression of such symptoms or to inhibit the development of such symptoms to any clinically measurable degree. The amount of the therapeutic agent effective to alleviate any specific disease symptom (also known as a "therapeutically effective amount") may vary depending on a variety of factors, such as the disease state, age and body weight of the patient, and the ability of the drug to produce a desired therapeutic effect in the patient. Whether a disease symptom has been alleviated can be evaluated by any clinical testing methods commonly used by doctors or other health care professionals to evaluate the severity or progression of the symptom. Although the embodiments of the present disclosure (e.g., treatment methods or products) may not be effective in alleviating all the target disease symptoms in every patient, as determined according to any statistical testing methods known in the art, such as Student t-test, chi-square test, Mann and Whitney's U test, Kruskal-Wallis test (H test), Jonckheere-Terpstra test, and Wilcoxon test, they should reduce the target disease symptoms in a statistically significant number of patients.

"Optional" or "optionally" means that the event or circumstance subsequently described may, but does not necessarily, occur, and this description includes instances where the event or circumstance occurs or does not occur. "And/or" should be taken as specific disclosure of each of the two specified features or components with or without the other. Thus, the term "and/or" as used in a phrase such as "A and/or B" in the present disclosure includes "A and B", "A or B", "A" (alone), and "B" (alone). Unless the context clearly requires otherwise, throughout the description and the claims, the words "comprise", "have", "include", and the like are to be understood in an inclusive sense as opposed to an exclusive or exhaustive sense; that is to say, in the sense of "including, but not limited to".

The "subject" or "patient" in the present disclosure means a mammal, particularly a primate, and especially a human.

The ordinal numbers "first", "second", "L1", "L2", etc. in the present disclosure are not intended to limit the quantity, level, rank and order, but are intended only to distinguish different elements, steps, and technical features.

### Examples

The present disclosure is further described with reference to the following examples, but these examples are not intended to limit the scope of the present disclosure. Experimental procedures without conditions specified in the examples of the present disclosure were generally conducted under conventional conditions such as those outlined in Antibodies: A Laboratory Manual and Molecular Cloning: A Laboratory Manual by Cold Spring Harbor Laboratory, or under conditions recommended by the manufacturers of the starting materials or commercial products. Reagents without specific origins indicated are commercially available conventional reagents.

### Example 1. Preparation of Serum Albumin Antigens and Proteins for Detection

### 1. Design and expression of human serum albumin:

Using human serum albumin (HSA) as a template, an amino acid sequence of a protein for detection with a deletion of some C-terminal amino acids was designed.

Full-length amino acid sequence of human serum albumin: 











 (SEQ ID NO: 1)
 (Note: the italicized and underlined part indicates a signal peptide; the italicized and bold part indicates a propeptide).

The albumin-FcRn binding region is distributed in the third domain of the albumin molecule; based on the molecular simulation structure, an albumin molecule with a deletion of some amino acids in the third domain at the C-terminus was designed (with an Avi-his tag fused at the C-terminus): 
DAHKSEVAHRFKDLGEENFKALVLIAFAQYLQQCPFEDHVKLVNEVTEFAKTCV
ADESAENCDKSLHTLFGDKLCTVATLRETYGEMADCCAKQEPERNECFLQHKD
DNPNLPRLVRPEVDVMCTAFHDNEETFLKKYLYEIARRHPYFYAPELLFFAKRY
KAAFTECCQAADKAACLLPKLDELRDEGKASSAKQRLKCASLQKFGERAFKA
WAVARLSQRFPKAEFAEVSKLVTDLTKVHTECCHGDLLECADDRADLAKYICEN
QDSISSKLKECCEKPLLEKSHCIAEVENDEMPADLPSLAADFVESKDVCKNYAE
AKDVFLGMFLYEYARRHPDYSVVLLLRLAKTYETTLEKCCAAADPHECYAKVF
 
DEFKPLVEEPQNLIKQNCELFEQLGEYKFQNALLVRYTKKVPQVSTPTLVEVSR
NLGKVGSKCCKHPEAKRMPCAEDYLSVVLNQLCVLHEKTPVSDRVTKCCTESL
VNRRPCFSALEVDETGGGGSGLNDIFEAQKIEWHEHHHHHH
(SEQ ID NO: 2)
 (Note: the italicized part indicates the Avi-his tag).
2. Full-length amino acid sequence of monkey serum albumin: 
MKWVTFISLLFLFSSAYSRGVFRRDTHKSEVAHRFKDLGEEHFKGLVLVA
FSQYLQQCPFEEHVKLVNEVTEFAKTCVADESAENCDKSLHTLFGDKLCT
VATLRETYGEMADCCAKQEPERNECFLQHKDDNPNLPPLVRPEVDVMCTA
FHDNEATFLKKYLYEVARRHPYFYAPELLFFAARYKAAFAECCQAADKAA
CLLPKLDELRDEGKASSAKQRLKCASLQKFGDRAFKAWAVARLSQKFPKA
EFAEVSKLVTDLTKVHTECCHGDLLECADDRADLAKYMCENQDSISSKLK
ECCDKPLLEKSHCLAEVENDEMPADLPSLAADYVESKDVCKNYAEAKDVF
LGMFLYEYARRHPDYSVMLLLRLAKAYEATLEKCCAAADPHECYAKVFDE
FQPLVEEPQNLVKQNCELFEQLGEYKFQNALLVRYTKKVPQVSTPTLVEV
SRNLGKVGAKCCKLPEAKRMPCAEDYLSVVLNRLCVLHEKTPVSEKVTKC
CTESLVNRRPCFSALELDEAYVPKAFNAETFTFHADMCTLSEKEKQVKKQ
TALVELVKHKPKATKEQLKGVMDNFAAFVEKCCKADDKEACFAEEGPKFV
AASQAALA
(SEQ ID NO: 3)
 (Note: the italicized and underlined part indicates a signal peptide; the italicized and bold part indicates a propeptide).
3. Full-length amino acid sequence of mouse serum albumin: 
MKWVTFLLLLFVSGSAFSRGVFRREAHKSEIAHRYNDLGEQHFKGLVLI
AFSQYLQKCSYDEHAKLVQEVTDFAKTCVADESAANCDKSLHTLFGDKLCAIP
NLRENYGELADCCTKQEPERNECFLQHKDDNPSLPPFERPEAEAMCTSFKENPT
TFMGHYLHEVARRHPYFYAPELLYYAEQYNEILTQCCAEADKESCLTPKLDGVK
EKALVSSVRQRMKCSSMQKFGERAFKAWAVARLSQTFPNADFAEITKLATDLTK
VNKECCHGDLLECADDRAELAKYMCENQATISSKLQTCCDKPLLKKAHCLSEV
EHDTMPADLPAIAADFVEDQEVCKNYAEAKDVFLGTFLYEYSRRHPDYSVSLLL
RLAKKYEATLEKCCAEANPPACYGTVLAEFQPLVEEPKNLVKTNCDLYEKLGEY
GFQNAILVRYTQKAPQVSTPTLVEAARNLGRVGTKCCTLPEDQRLPCVEDYLSA
ILNRVCLLHEKTPVSEHVTKCCSGSLVERRPCFSALTVDETYVPKEFKAETFTFH
SDICTLPEKEKQIKKQTALAELVKHKPKATAEQLKTVMDDFAQFLDTCCKAADK
DTCFSTEGPNLVTRCKDALA
(SEQ ID NO: 4)
 (Note: the italicized and underlined part indicates a signal peptide; the italicized and bold part indicates a propeptide).
4. Full-length amino acid sequence of rat serum albumin: 
MKWVTFLLLLFISGSAFSRGVFRREAHKSEIAHRFKDLGEQHFKGLVLIAFSQYLQ
KCPYEEHIKLVQEVTDFAKTCVADENAENCDKSIHTLFGDKLCAIPKLRDNYGE
 
LADCCAKQEPERNECFLQHKDDNPNLPPFQRPEAEAMCTSFQENPTSFLGHYLH
EVARRHPYFYAPELLYYAEKYNEVLTQCCTESDKAACLTPKLDAVKEKALVAAV
RQRMKCSSMQRFGERAFKAWAVARMSQRFPNAEFAEITKLATDVTKINKECCH
GDLLECADDRAELAKYMCENQATISSKLQACCDKPVLQKSQCLAEIEHDNIPAD
LPSIAADFVEDKEVCKNYAEAKDVFLGTFLYEYSRRHPDYSVSLLLRLAKKYEA
TLEKCCAEGDPPACYGTVLAEFQPLVEEPKNLVKTNCELYEKLGEYGFQNAVLV
RYTQKAPQVSTPTLVEAARNLGRVGTKCCTLPEAQRLPCVEDYLSAILNRLCVL
HEKTPVSEKVTKCCSGSLVERRPCFSALTVDETYVPKEFKAETFTFHSDICTLPD
KEKQIKKQTALAELVKHKPKATEDQLKTVMGDFAQFVDKCCKAADKDNCFAT
EGPNLVARSKEALA
(SEQ ID NO: 5)
 (Note: the italicized and underlined part indicates a signal peptide; the italicized and bold part indicates a propeptide).

### Example 2. Screening of Antibodies Specifically Binding to Human Serum Albumin (HSA)

### 1. Camel immunization

A natural Bactrian camel from Inner Mongolia was immunized with serum albumin extracted from commercial human blood (Sigma, Cat No. 126658). Then, 5 mL of pre-immunization camel blood was taken, and the serum was separated. Complete or incomplete Freund's adjuvant was mixed with the antigen in a volume ratio of 1:1, and then the camel was subjected to subcutaneous multi-site immunization with the mixture (at an immunization dose of 100 µg of protein per camel each time). Boost immunization was performed once every two weeks, and the titer was determined after four immunizations. A plate (Costar, Cat. No. 9018) was coated with 5 µg/mL human serum albumin (HSA) at 100 µL/well and left to stand overnight at 4 °C. The next day, the plate was washed three times with PBST (0.05% Tween 20) at 300 µL/well, and then blocked with 4% skim milk powder at 37 °C for 2 h. After washing, serum from the immunized camel was diluted in a gradient and added, and the mixture was incubated at 37 °C for 1 h. The negative control consisted of pre-immunization serum diluted in the same gradient and a blank PBS solution. After the incubation was completed, the plate was washed three times with PBST, and then a horseradish peroxidase-labeled goat anti-camel Fc polyclonal antibody (Thermo, Cat No. A16060, diluted at 1:5000) was added. The mixture was incubated at 37 °C for 1 h. The plate was washed again, and then a TMB chromogenic solution was added for chromogenic reaction. The reaction was terminated with 1 M sulfuric acid, and the absorbance values at a wavelength of OD450 nm were taken using a SpectraMax M5 microplate reader. After a 1:51200 dilution, the titer was detected. The titer was qualified, and peripheral blood from the camel was collected for library construction.

### 2. Phage library construction

100-200 mL of the peripheral blood from the camel was taken, and peripheral blood lymphocytes (PBMCs) were separated using the Ficoll method, resulting in a cell count of 1.2 × 10⁸ cells. Trizol reagent was added for resuspension (1 × 10⁷ cells/mL Trizol) to lyse the cells, and the mixture was left to stand on ice for 5 min and then centrifuged at 13000 rpm for 3 min. The supernatant was collected, and the pellet was discarded. Then, 1/5 volume of chloroform was added, and the mixture was shaken vigorously for 30-60 s, then left to stand in an ice bath for 2 min, and centrifuged at 13000 rpm for 10 min. The upper aqueous phase was pipetted and transferred into a new 1.5 mL tube. Subsequently, an equal volume of isopropanol was added, and the mixture was mixed well, left to stand at -20 °C for 30 min, and then centrifuged at 13000 rpm for 10 min. The supernatant was removed, and the pellet was retained. The pellet was then washed with pre-cooled 75% ethanol and left to stand at room temperature for 5-10 min. Then, 600 µL of deionized water from which RNase was removed was added for re-dissolution to obtain RNA, which was then reverse-transcribed to obtain cDNA for the construction of a phage library.

### 3. Phage library screening

The HSA protein with the deletion of some amino acids in the third domain at the C-terminus (also known as truncated HSA, with the sequence set forth in SEQ ID NO: 2) was expressed in HEK293 cells and purified, and this protein was used for phage library screening. Antibodies with affinity for HSA were obtained by screening.

10 µg of the aforementioned truncated HSA-avi-biotin protein was bound to 1 mg of Dynabeads M-280 Streptavidin (Invitrogen, Cat No. 11206D). The beads were incubated at room temperature for one hour, then washed 3 times with 1× PBS, and blocked with 2% skim milk at room temperature for 2 h. A camelid heavy-chain single-domain antibody phage display library was then added, and the mixture was allowed to react at room temperature for 1 h. The mixture was washed 9 times with a PBST (0.05% Tween-20) solution to remove unbound phages. Phages specifically binding to truncated HSA were eluted with 1 mg/mL trypsin and then used to infect *E. coli* TG1 in the logarithmic growth phase. Then, phages were generated and purified for the next round of screening. After repeating the same screening process for 1-2 rounds, the positive clone sequences were enriched.

### 4. Phage ELISA identification

Monoclonal colonies were picked from the clones obtained from the final round of screening and then packaged into phage single-chain antibodies for phage ELISA testing. ELISA plates were coated overnight with 2 µg/mL human, monkey and mouse serum albumins (HSA, CySA, and mSA), respectively, and then blocked with 4% skim milk at 37 °C for 1 h. Subsequently, phage supernatant blocked with 2% skim milk was added, and the mixture was allowed to react at room temperature for 1 h. The plates were then washed 3 times with PBST (0.05% Tween 20), and anti-M13 HRP was added for detection. Clones with OD450 values greater than 0.5 for all three albumins in the ELISA binding test were sequenced, resulting in 50 specific sequences. Antibodies that showed high OD450 values only for both human and monkey serum albumins were sequenced, resulting in 7 additional new specific sequences. Through the phage screening, 57 new specific sequences were obtained.

### Example 3. Construction of Antibody Expression Vectors in Mammalian Cells

The 57 specific sequences obtained from the phage library screening in Example 2 were categorized and organized, and 19 of them were cloned. The phage supernatant ELISA binding of these 19 anti-HSA antibodies was tested. The OD450 values of clones #7 and #9 are shown in Table 1.

**Table 1. Phage supernatant ELISA test results for anti-HSA antibodies**

| No. | OD450 for human HSA | OD450 for mouse SA | OD450 for monkey SA |
|---|---|---|---|
| #7aHSA | 1.6929 | 1.1185 | 1.7477 |
| #9aHSA | 1.5231 | 1.0871 | 1.8161 |

> #7aHSA 
HVQLVESGGGSVQAGGSLRVSCVASGNTYLTSCMGWFRQAPGKEREGVASIYTVGG
STFYTNSVKGRFTISRDNAKNTVYLQMNSLQPEDTAMYYCAAGSPARCRLRDPTTF
DYWGQGTQVTVSS (SEQ ID NO: 6)
> #9aHSA

DVQLVESGGGSVQAGGSLRLSCAAAEYTYLNSCLGWFRQAPGKEREGVATIYMVG
GSTFYADSVKGRFTISRDNAKNTVYLMMNSLKPEDTAMYYCAAGSPARCLRRDPTT
FNYWGQGTQVTVSS (SEQ ID NO: 7).

In the above sequences SEQ ID NOs: 6 and 7, the regions are arranged in the following order FR1-CDR1-FR2-CDR2-FR3-CDR3-FR4. In the sequences, the italicized parts indicate the FR sequences, and the underlined parts indicate the CDR1, CDR2 and CDR3 sequences, respectively. The CDR sequences of the anti-HSA antibodies provided by the present disclosure according to Kabat numbering are summarized in Table 2.

**Table 2. CDR sequences of anti-HSA antibodies (Kabat numbering scheme)**

| Antibody No. | CDR sequence and sequence No. | | |
|---|---|---|---|
| #7aHSA | CDR1 | TSCMG | SEQ ID NO: 8 |
| | CDR2 | SIYTVGGSTFYTNSVKG | SEQ ID NO: 9 |
| | CDR3 | GSPARCRLRDPTTFDY | SEQ ID NO: 10 |
| #9aHSA | CDR1 | NSCLG | SEQ ID NO: 11 |
| | CDR2 | TIYMVGGSTFYADSVKG | SEQ ID NO: 12 |
| | CDR3 | GSPARCLRRDPTTFNY | SEQ ID NO: 13 |

Thus, we provided the CDRs of the anti-HSA antibodies as follows:
a CDR1 of X₁SCX₂G, wherein X₁ is T or N, and X₂ is M or L;
a CDR2 of X₃IYX₄VGGSTFYX₅X₆SVKG (SEQ ID NO: 58), wherein X₃ is S or T, X₄ is T or M, X₅ is T or A, and X₆ is N or D; and
a CDR3 of GSPARCX₇X₈RDPTTFX₉Y (SEQ ID NO: 59), wherein X₇ is R or L, X₈ is L or R, and X₉ is D or N.

The VHH sequences were each fused to human IgG1-Fc (CH2-CH3) and constructed into a PTT5 expression vector; the sequence of the linked human IgG1-Fc is shown below:
> Human IgG1-Fc

EPKSCDKTHTCPPCPAPELLGGPSVFLFPPKPKDTLMISRTPEVTCVVVDVSHED
PEVKFNWYVDGVEVHNAKTKPREEQYNSTYRVVSVLTVLHQDWLNGKEYKC
KVSNKALPAPIEKTISKAKGQPREPQVYTLPPSRDELTKNQVSLTCLVKGFYPSDI
AVEWESNGQPENNYKTTPPVLDSDGSFFLYSKLTVDKSRWQQGNVFSCSVMHE
ALHNHYTQKSLSLSPGK (SEQ ID NO: 14)

The following are full sequences of the VHH sequences fused to the human Fc (CH2-CH3) segment, where the single underlined parts indicate the sequence of the human IgG1-Fc (CH2-CH3) segment (SEQ ID NO: 14), and the double underlined parts indicate the sequence of the linker. The full sequences are as follows:
> #7aHSA-IgG1Fc: 
HVQLVESGGGSVQAGGSLRVSCVASGNTYLTSCMGWFRQAPGKEREGVASIYT
VGGSTFYTNSVKGRFTISRDNAKNTVYLQMNSLQPEDTAMYYCAAGSPARCRL
RDPTTFDYWGQGTQVTVSSASGSEPKSCDKTHTCPPCPAPELLGGPSVFLFPPKP
KDTLMISRTPEVTCVVVDVSHEDPEVKFNWYVDGVEVHNAKTKPREEQYNST
YRVVSVLTVLHQDWLNGKEYKCKVSNKALPAPIEKTISKAKGQPREPQVYTLPP
SRDELTKNQVSLTCLVKGFYPSDIAVEWESNGQPENNYKTTPPVLDSDGSFFLYS
KLTVDKSRWQQGNVFSCSVMHEALHNHYTQKSLSLSPGK (SEQ ID NO: 15)
> #9aHSA-IgG1Fc:

DVQLVESGGGSVQAGGSLRLSCAAAEYTYLNSCLGWFRQAPGKEREGVATIYM
VGGSTFYADSVKGRFTISRDNAKNTVYLMMNSLKPEDTAMYYCAAGSPARCLR
RDPTTFNYWGQGTQVTVSSASGSEPKSCDKTHTCPPCPAPELLGGPSVFLFPPKP
KDTLMISRTPEVTCVVVDVSHEDPEVKFNWYVDGVEVHNAKTKPREEQYNST
YRVVSVLTVLHQDWLNGKEYKCKVSNKALPAPIEKTISKAKGQPREPQVYTLPP
SRDELTKNQVSLTCLVKGFYPSDIAVEWESNGQPENNYKTTPPVLDSDGSFFLYS
KLTVDKSRWQQGNVFSCSVMHEALHNHYTQKSLSLSPGK (SEQ ID NO: 16).

### Example 4. Expression, Screening, and Detection of Anti-HSA Antibodies

The #7aHSA and #9a anti-HSA antibodies, fused with human IgG1-Fc, were separately transiently expressed in HEK293 cells and subjected to ProteinA affinity purification. Then, detection was performed by an ELISA binding assay and a Biacore protein interaction experiment.

The *in vitro* binding ability of the #7aHSA and #9a anti-HSA antibodies to human, monkey and mouse serum albumins was detected through the ELISA binding assay. The antigens used in the assay were human serum albumin (Sigma, Cat No. 126658), monkey serum albumin (Abcam, Cat No. ab184894) and mouse serum albumin (Abcam, Cat No. ab183228). PBS was used as a negative control, and ALB8 fused with human IgG1-Fc (the variable region sequence is from the ALB8 sequence in Ablynx patent publication WO 2008/028977 A2) was used as a positive control, with the following sequence:
> ALB8-IgG1-Fc 
EVQLVESGGGLVQPGNSLRLSCAASGFTFSSFGMSWVRQAPGKGLEWVSSISGS
GSDTLYADSVKGRFTISRDNAKTTLYLQMNSLRPEDTAVYYCTIGGSLSRSSQGT
LVTVSSASGSEPKSCDKTHTCPPCPAPELLGGPSVFLFPPKPKDTLMISRTPEVTC
VVVDVSHEDPEVKFNWYVDGVEVHNAKTKPREEQYNSTYRVVSVLTVLHQD
WLNGKEYKCKVSNKALPAPIEKTISKAKGQPREPQVYTLPPSRDELTKNQVSLT
CLVKGFYPSDIAVEWESNGQPENNYKTTPPVLDSDGSFFLYSKLTVDKSRWQQG
NVFSCSVMHEALHNHYTQKSLSLSPGK (SEQ ID NO: 17)
 (Note: the single underlined part indicates the Fc region of IgG1, and the double underlined part indicates the linker region).

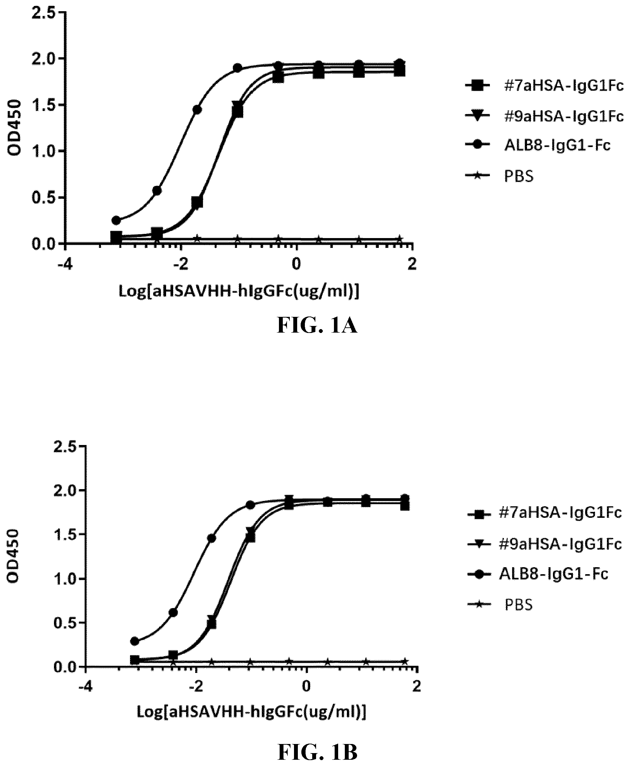

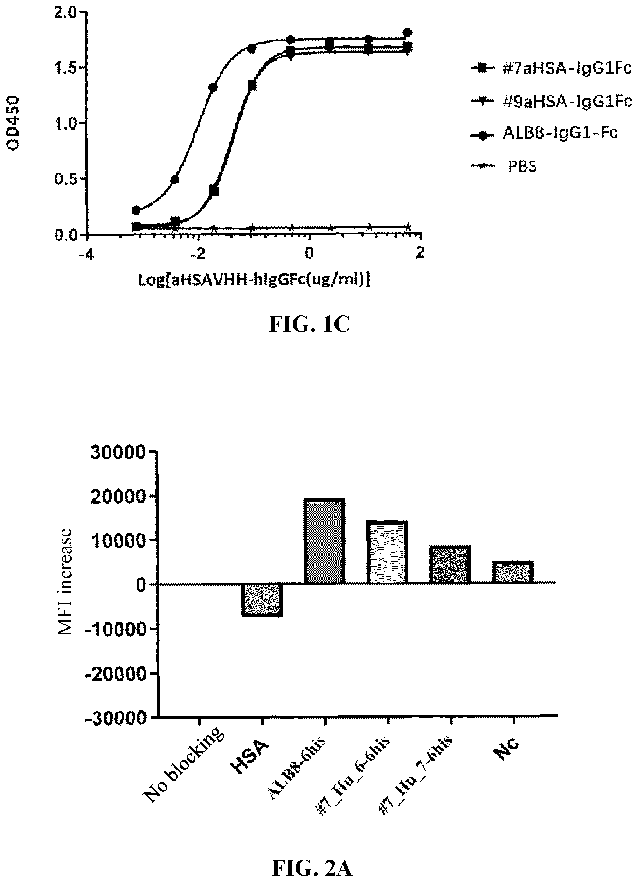

Human, monkey and mouse albumins were each diluted to 1 µg/mL with a PBS buffer (pH 7.4) and added to a 96-well microplate (Corning, Cat No. 9018) at a volume of 100 µL/well. The plate was left to stand overnight at 4 °C for 16-20 h. After the liquid was discarded, the plate was washed three times with PBST (pH 7.4, 0.05% Tween-20) buffer, and then a 2% skim milk powder blocking solution diluted with a PBS buffer was added (300 µL/well). The mixture was incubated at 37 °C for 1 h for blocking. After the blocking was completed, the blocking solution was discarded, and the plate was washed 3 times with PBST buffer. Then, the anti-HSA antibody at an initial concentration of 2 µg/mL was added, and the mixture was subjected to three-fold dilution with a PBS buffer across 11 gradients, and then incubated at 25 °C for 1 h. After the incubation was completed, the reaction liquid in the plate was discarded, and the plate was washed 3 times with PBST. Then, 100 µL of an HRP-labeled goat anti-human IgGFc secondary antibody (Jackson immu, 109-035-190) (diluted at 1:5000) was added to each well, and the mixture was incubated at 25 °C for 1 h. After the plate was washed 3 times with PBST, 100 µL of a TMB chromogenic substrate was added for chromogenic reaction. The reaction was terminated with 1 M sulfuric acid, and the absorbance values at OD450 nm were taken using a SpectraMax M5 microplate reader. GraphPad prism was used to plot the absorbance values and antibody concentrations by 4-parameter fitting, with the results shown in FIGs. 1A to 1C. The EC₅₀ values for the binding of the antibodies to the antigens were calculated, and the results are shown in Table 3.

**Table 3. EC₅₀ values (µg/mL) for ELISA binding of anti-HSA antibodies to human, monkey and mouse albumins**

| Antibody No. | EC₅₀ for binding to HSA | EC₅₀ for binding to monkey SA | EC₅₀ for binding to mouse SA |
|---|---|---|---|
| #7aHSA-IgG1Fc | 0.04552 | 0.04256 | 0.04502 |
| #9aHSA-IgG1Fc | 0.04601 | 0.03865 | 0.04116 |
| Positive control (ALB8-IgG1Fc) | 0.009926 | 0.010134 | 0.01061 |
| Negative control (PBS) | / | / | / |

| | | | |
|---|---|---|---|
| (Note: "/" indicates no detectable binding) | | | |

Additionally, the affinity constants of the anti-HSA antibodies fused with human IgG1Fc for human, monkey and mouse albumins (HSA, CySA, and mSA) were also determined using a Biacore T200 (GE Healthcare) instrument. Firstly, protein A was covalently coupled onto a CM5 S series chip (GE, Cat. 29-1049-88) using an amino coupling kit (GE, Cat. BR-1000-50), and the anti-HSA antibody was captured onto the protein A-coupled chip through affinity. Then, different concentrations of human, monkey and mouse albumins were flowed over the surface of the chip, and the reaction signals were detected in real-time using the Biacore instrument to obtain the association and dissociation curves. Finally, fitting was performed with a Langmuir 1:1 binding model using BIAevaluation version 4.1, GE software to obtain the affinity constants. The buffer used in the experiment was an HBS-EP solution (10 mM HEPES, 150 mM NaCl, 3 mM EDTA, 0.05% P20, pH 7.4, GE, Cat. # BR-1006-69). After the end of each cycle, the chip was regenerated using glycine-hydrochloric acid (pH 1.5) (GE, Cat. # BR-1003-54). The affinity results are shown in Table 4.

**Table 4. Results for the affinity of anti-HSA antibodies for human, monkey and mouse albumins**

| Antibody No. | Antigen | Ka (1/Ms) | Kd (1/s) | K_{D} (M) |
|---|---|---|---|---|
| #7aHSA-IgG1Fc | Human HSA | 4.29E+04 | 7.55E-05 | 1.76E-09 |
| | Monkey SA | 4.49E+04 | 5.70E-05 | 1.27E-09 |
| | Mouse SA | 4.66E+04 | 3.47E-04 | 7.44E-09 |
| #9aHSA-IgG1Fc | Human HSA | 8.43E+04 | 2.64E-05 | 3.13E-10 |
| | Monkey SA | 8.92E+04 | 4.40E-05 | 4.93E-10 |
| | Mouse SA | 2.21E+09 | 3.10E+01 | 1.40E-08 |

### Example 5. Humanization of Anti-HSA Antibodies

By three-dimensional structure homologous modeling of the selected specific anti-HSA antibody molecule #7aHSA, in combination with the results of comparison with a V-base human germline sequence database and an IMGT human antibody heavy chain variable region germline gene database, the heavy chain variable region germline gene IGHV3-23 having high homology with the selected antibody was chosen as a template, and CDRs of the camelid-derived single-domain antibody were grafted into the corresponding human template, forming a variable region sequence in the order of FR1-CDR1-FR2-CDR2-FR3-CDR3-FR4. Three-dimensional structure modeling and analysis were further performed on the grafted single-domain antibody. Back mutations were performed on the specific sites in the FRs that affected the structure and morphology of the CDRs. The engineered antibodies had higher stability. The amino acid residues were identified using the Kabat numbering scheme and annotated. Among these antibodies, #7_Hu-9 and #7_Hu-10 retained the human germline sequences to a greater extent in their FRs, resulting in fewer immunogenic mutations.

The design of the back mutation positions and mutation modes is shown in Table 5.

**Table 5. Back mutation positions and mutation modes**

| Antibody No. | Back mutation |
|---|---|
| #7_Hu_1 | F27N; F29Y; S30L; V37F; G44E; L45R; W47G |
| #7_Hu_2 | L20V; F27N; F29Y; S30L; V37F; G44E; L45R; W47G |
| #7_Hu_3 | A23V; F27N; F29Y; S30L; V37F; G44E; L45R; W47G |
| #7_Hu_4 | F27N; F29Y; S30L; V37F; G44E; L45R; W47G; S74A |
| #7_Hu_5 | F27N; F29Y; S30L; V37F; G44E; L45R; W47G; L78V |
| #7_Hu_6 | F27N; F29Y; S30L; V37F; G44E; L45R; W47G; S49A |
| #7_Hu_7 | F27N; F29Y; S30L; V37F; G44E; L45R; W47G; R83Q; A84P |
| #7_Hu_8 | F27N; F29Y; S30L; V37F; G44E; L45R; W47G; A84P |
| #7_Hu_9 | S30L; V37F; G44E; L45R; W47G; A84P |
| #7_Hu-10 | S30L; V37F; G44E; L45R; W47G |

The specific humanized sequences obtained are as follows, numbered according to the Kabat numbering scheme, with the underlined parts indicating the CDRs:
> #7_Hu_1 
EVQLLESGGGLVQPGGSLRLSCAASGNTYLTSCMGWFRQAPGKEREGVSSIYT
VGGSTFYTNSVKGRFTISRDNSKNTLYLQMNSLRAEDTAVYYCAAGSPARCRLR
DPTTFDYWGQGTLVTVSS (SEQ ID NO: 18)
> #7_Hu_2 
EVQLLESGGGLVQPGGSLRVSCAASGNTYLTSCMGWFRQAPGKEREGVSSIYT
VGGSTFYTNSVKGRFTISRDNSKNTLYLQMNSLRAEDTAVYYCAAGSPARCRLR
DPTTFDYWGQGTLVTVSS (SEQ ID NO: 19)
> #7_Hu**_**3 
EVQLLESGGGLVQPGGSLRLSCVASGNTYLTSCMGWFRQAPGKEREGVSSIYT
VGGSTFYTNSVKGRFTISRDNSKNTLYLQMNSLRAEDTAVYYCAAGSPARCRLR
DPTTFDYWGQGTLVTVSS (SEQ ID NO: 20)
> #7_Hu_4 
EVQLLESGGGLVQPGGSLRLSCAASGNTYLTSCMGWFRQAPGKEREGVSSIYTV
GGSTFYTNSVKGRFTISRDNAKNTLYLQMNSLRAEDTAVYYCAAGSPARCRLR
DPTTFDYWGQGTLVTVSS (SEQ ID NO: 21)
> #7_Hu**_**5 
EVQLLESGGGLVQPGGSLRLSCAASGNTYLTSCMGWFRQAPGKEREGVSSIYTV
GGSTFYTNSVKGRFTISRDNSKNTVYLQMNSLRAEDTAVYYCAAGSPARCRLR
DPTTFDYWGQGTLVTVSS (SEQ ID NO: 22)
> #7_Hu**_**6 
EVQLLESGGGLVQPGGSLRLSCAASGNTYLTSCMGWFRQAPGKEREGVASIYT
VGGSTFYTNSVKGRFTISRDNSKNTLYLQMNSLRAEDTAVYYCAAGSPARCRLR
DPTTFDYWGQGTLVTVSS (SEQ ID NO: 23)
> #7_Hu**_**7 
EVQLLESGGGLVQPGGSLRLSCAASGNTYLTSCMGWFRQAPGKEREGVSSIYTV
GGSTFYTNSVKGRFTISRDNSKNTLYLQMNSLQPEDTAVYYCAAGSPARCRLRD
PTTFDYWGQGTLVTVSS (SEQ ID NO: 24)
> #7_Hu**_**8 
EVQLLESGGGLVQPGGSLRLSCAASGNTYLTSCMGWFRQAPGKEREGVSSIYTV
GGSTFYTNSVKGRFTISRDNSKNTLYLQMNSLRPEDTAVYYCAAGSPARCRLRD
PTTFDYWGQGTLVTVSS (SEQ ID NO: 25)
> #7_Hu_9 
EVQLLESGGGLVQPGGSLRLSCAASGFTFLTSCMGWFRQAPGKEREGVSSIYTV
GGSTFYTNSVKGRFTISRDNSKNTLYLQMNSLRPEDTAVYYCAAGSPARCRLRD
PTTFDYWGQGTLVTVSS (SEQ ID NO: 26)
> #7**_**Hu_10

EVQLLESGGGLVQPGGSLRLSCAASGFTFLTSCMGWFRQAPGKEREGVSSIYTV
GGSTFYTNSVKGRFTISRDNSKNTLYLQMNSLRAEDTAVYYCAAGSPARCRLRD
PTTFDYWGQGTLVTVSS (SEQ ID NO: 27).

Using the method in Example 5, fusion proteins of the humanized anti-HSA antibodies with the Fc (CH2-CH3) segment of hIgG1 or a 6His tag were constructed. The single underlined parts indicate the sequence of the hIgG1-Fc (CH2-CH3) segment (SEQ ID NO: 14), the italicized parts indicate the sequence of the 6His tag, and the double underlined parts indicate the sequences of the linkers. The sequences of the protein are as follows (using #7_hu_1-hIgG1Fc and #7_hu_10-6His as examples; the other humanized anti-HSA antibodies were defined in the same way). For the antibody numbering scheme, #7_hu_10-6His means that #7_Hu_10 (SEQ ID NO: 26) had "GGGGSGGGS*HHHHHH"* fused at the C-terminus, and the other antibodies were defined in the same way.
> #7_hu_1-hIgG1Fc: 
EVQLLESGGGLVQPGGSLRLSCAASGNTYLTSCMGWFRQAPGKEREGVSSIYTV
GGSTFYTNSVKGRFTISRDNSKNTLYLQMNSLRAEDTAVYYCAAGSPARCRLRD
PTTFDYWGQGTLVTVSSASGSEPKSCDKTHTCPPCPAPELLGGPSVFLFPPKPKD
TLMISRTPEVTCVVVDVSHEDPEVKFNWYVDGVEVHNAKTKPREEQYNSTYRV
VSVLTVLHQDWLNGKEYKCKVSNKALPAPIEKTISKAKGQPREPQVYTLPPSRD
ELTKNQVSLTCLVKGFYPSDIAVEWESNGQPENNYKTTPPVLDSDGSFFLYSKLT
VDKSRWQQGNVFSCSVMHEALHNHYTQKSLSLSPGK (SEQ ID NO: 28)
> #7_Hu**_**10-6His 
EVQLLESGGGLVQPGGSLRLSCAASGFTFLTSCMGWFRQAPGKEREGVSSIYTV
GGSTFYTNSVKGRFTISRDNSKNTLYLQMNSLRAEDTAVYYCAAGSPARCRLRD
PTTFDYWGQGTLVTVSSGGGGSGGGSHHHHHH (SEQ ID NO: 29)
> ALB8-6his

EVQLVESGGGLVQPGNSLRLSCAASGFTFSSFGMSWVRQAPGKGLEWVSSISGS
GSDTLYADSVKGRFTISRDNAKTTLYLQMNSLRPEDTAVYYCTIGGSLSRSSQGT
LVTVSSGGGGSGGGSHHHHHH (SEQ ID NO: 30).

HEK293 cells were transiently transfected with plasmids. After 5 days of cell culture, the expression supernatants were collected and centrifuged at 4000 rpm for 15 min to remove the cells. The resulting supernatants were harvested, filtered using a 0.45 µM filter, and then purified and isolated using conventional methods in the art, such as a Protein A column or a nickel column. Analysis showed that the anti-HSA antibodies of the present disclosure were obtained.

### Example 6. Assay for Affinity of Humanized Anti-HSA Antibodies for Human, Monkey and Mouse Serum Albumins

The affinity constants of the 6His-tagged humanized anti-HSA antibodies for human, monkey and mouse albumins were determined using a Biacore T200 (GE Healthcare) instrument. Firstly, amino coupling was performed on a CM5 S series chip (GE, Cat. 29-1049-88) by using an amino coupling kit (GE, Cat. BR-1000-50) and a His capture kit (GE, Cat. 28-9950-56) according to the kit manuals. The surface response value reached about 10000 RU. The chip was then blocked with ethanolamine for later use. The 6His-tagged humanized anti-HSA antibodies were each captured onto the anti-his chip through affinity. Then, different concentrations of human, monkey and mouse albumins were flowed over the surface of the chip, and the reaction signals were detected in real-time using the Biacore instrument to obtain the association and dissociation curves. Finally, fitting was performed with a Langmuir 1:1 binding model using BIAevaluation version 4.1, GE software to obtain the affinity constants. The buffer used in the experiment was an HBS-EP solution (10 mM HEPES, 150 mM NaCl, 3 mM EDTA, 0.05% P20, pH 7.4, GE, Cat. # BR-1006-69). After the end of each cycle, the chip was regenerated using glycine-hydrochloric acid (pH 1.5) (GE, Cat. # BR-1003-54). The affinity results for some of the humanized antibodies are shown in Table 6. The results show that the affinity of the antibodies obtained by screening of the present disclosure for HSA was comparable to or slightly better than that of the positive control.

**Table 6. The affinity of humanized anti-HSA antibodies for human, monkey and mouse albumins**

| Antibody No. | | ALB8-6his | #7_hu_1-6his | #7_hu_5-6his | #7_hu_6-6his | #7_hu_7-6his | #7_hu_10-6his |
|---|---|---|---|---|---|---|---|
| Human HSA | ka (1/Ms) | 2.62E+04 | 3.60E+04 | 3.55E+04 | 3.71E+04 | 3.74E+04 | 3.01E+04 |
| | kd (1/s) | 1.72E-03 | 6.53E-04 | 5.34E-04 | 6.40E-04 | 6.68E-04 | 3.47E-04 |
| | K_{D} (M) | 6.57E-08 | 1.81E-08 | 1.51E-08 | 1.73E-08 | 1.79E-08 | 1.15E-08 |
| Monkey SA | ka (1/Ms) | 2.30E+04 | 3.30E+04 | 3.31E+04 | 3.41E+04 | 3.49E+04 | 1.76E+04 |
| | kd (1/s) | 1.46E-03 | 5.54E-04 | 4.80E-04 | 5.45E-04 | 5.47E-04 | 5.45E-04 |
| | K_{D} (M) | 6.34E-08 | 1.68E-08 | 1.45E-08 | 1.60E-08 | 1.57E-08 | 3.10E-08 |
| Mouse SA | ka (1/Ms) | 2.25E+04 | 4.55E+04 | 3.93E+04 | 4.83E+04 | 4.61E+04 | 5.97E+04 |
| | kd (1/s) | 1.95E-02 | 2.62E-02 | 2.60E-02 | 2.81E-02 | 2.29E-02 | 1.96E-02 |
| | K_{D} (M) | 8.67E-07 | 5.77E-07 | 6.62E-07 | 5.82E-07 | 4.97E-07 | 3.28E-07 |

The anti-HSA antibody 7_Hu_10-6his was transiently expressed in HEK293E cells. After affinity purification with a nickel column, the affinity constants of the antibody for human, monkey, mouse, rat, dog and rabbit albumins were determined using a Biacore T200 (GE Healthcare) instrument. Firstly, amino coupling was performed on a CM5 S series chip (GE, Cat. 29-1049-88) by using an amino coupling kit (GE, Cat. BR-1000-50) and a His capture kit (GE, Cat. 28-9950-56) according to the kit manuals. The surface response value reached about 10000 RU. The chip was then blocked with ethanolamine for later use. The 6His-tagged humanized anti-HSA antibodies were each captured onto the anti-his chip through affinity. Then, different concentrations of human, monkey, mouse, rat, dog and rabbit albumins were flowed over the surface of the chip, and the reaction signals were detected in real-time using the Biacore instrument to obtain the association and dissociation curves. Finally, fitting was performed with a Langmuir 1:1 binding model using BIAevaluation version 4.1, GE software to obtain the affinity constants. The buffer used in the experiment was an HBS-EP solution (10 mM HEPES, 150 mM NaCl, 3 mM EDTA, 0.05% P20, pH 7.4, GE, Cat. # BR-1006-69). After the end of each cycle, the chip was regenerated using glycine-hydrochloric acid (pH 1.5) (GE, Cat. # BR-1003-54). The affinity results are shown in Table 7.

**Table 7. The affinity of #7_Hu_10-6his for albumins from different species**

| Sample | ka (1/Ms) | kd (1/s) | K_{D} (M) |
|---|---|---|---|
| Human HSA | 6.47E+04 | 6.83E-04 | 1.06E-08 |
| Monkey SA | 4.79E+04 | 6.97E-04 | 1.45E-08 |
| Mouse SA | 3.89E+04 | 2.54E-02 | 6.53E-07 |
| Rat SA | 2.92E+04 | 4.61E-03 | 1.58E-07 |
| Dog SA | 6.17E+04 | 2.11E-02 | 3.42E-07 |
| Rabbit SA | No detectable binding | | |

### Example 7. Effects of Anti-HSA Antibodies on Binding of FcRn to Biotin-HSA in Cells Stably Expressing hFcRn

The blocking effects of the anti-HSA antibodies on the binding of HSA to FcRn were detected by measuring the change in fluorescence of randomly biotinylated HSA bound to the surface of HEK293 cells stably expressing an hFcRn mutant (293-hFcRn-mut cells) caused by the antibodies. In this experiment, HEK293 cells stably expressing an hFcRn mutant (L320A, L321A) were used, where the hFcRn mutant could be well localized and maintained on the cell membrane surface. The cells expressing the hFcRn mutant were co-incubated with different concentrations of anti-HSA antibody and Biotin-HSA, followed by addition of an SA-FITC secondary antibody for incubation. The degree of reduction in the fluorescence signal (MFI) of the secondary antibody was used as a measure of the blocking effects of the anti-HSA antibodies on the binding of biotin-HSA to FcRn. The specific experimental procedures were as follows:

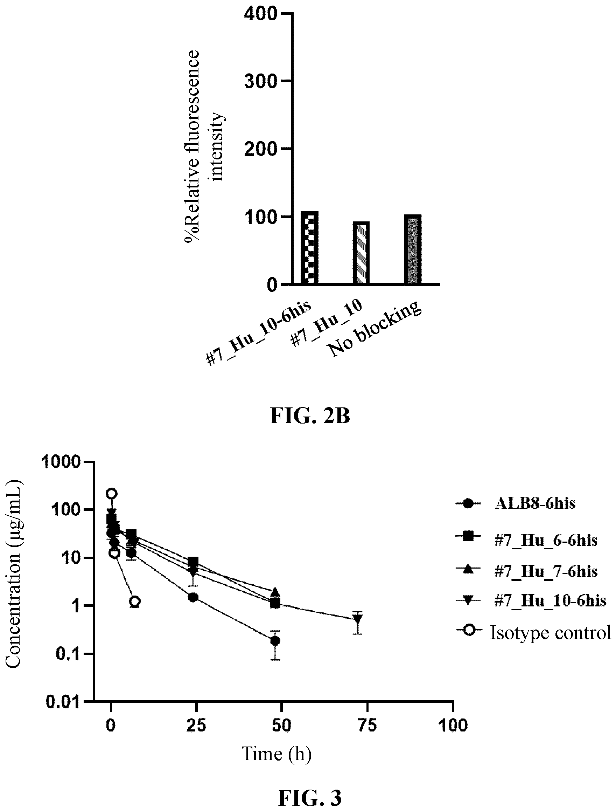

293-hFcRn-mut cells were washed twice with PBS (pH 5.5) and then added to a U-bottom 96-well plate at 5 × 10⁵ cells per well. After centrifugation and removal of the supernatant, 90 µL of anti-HSA antibody diluted with PBS (pH 5.5) and 10 µL of biotin-HSA (at a final concentration of 250 µg/mL) were added to each well, and the mixture was incubated on ice for 40 min. The plate was then washed twice with 200 µL of PBS (pH 5.5), and 100 µL of a streptavidin-FITC secondary antibody (eBioscience, Cat No. 11-4317-87) diluted with PBS (pH 5.5) at a 1:200 dilution ratio was added to each well. The mixture was incubated on ice for 40 min. The plate was then washed twice with 200 µL of PBS (pH 5.5), and 200 µL of PBS (pH 5.5) was added to each well. The fluorescence values were measured using a flow cytometer (BD Biosciences, BD Accuri C6) with the FL1 channel, and the data was analyzed using the Flowjo software. The analysis results were plotted using the Prism6 software. The assay results for the inhibitory effects of some of the antibodies are shown in FIG. 2A and FIG. 2B.

Analysis of results: Whether the binding of the anti-HSA antibodies to HSA blocked the binding of HSA to FcRn was determined based on the fluorescence signal values (MFI); a decrease in fluorescence signal indicates that the binding was blocked.

As shown in FIG. 2A, we used the biotin-HSA + FcRn stably transfected cells + SA-FITC group (non-blocking group) as a baseline, the biotin-HSA + HSA + FcRn stably transfected cells + SA-FITC group (HSA self-competition group) as a positive control, and Nc as an isotype control antibody. The results show that the anti-HSA antibodies #7_Hu_6-6his and #7_Hu_7-6his of the present disclosure did not block the binding of HSA to FcRn.

As shown in FIG. 2B, #7_Hu_10-6his and #7_Hu_10 did not block the binding of HSA to FcRn (detected at a concentration of 1 mg/mL).

This indicates that the anti-HSA antibodies of the present disclosure did not affect the pH-dependent binding of FcRn to HSA, and could effectively maintain the FcRn-mediated serum albumin recycling mechanism.

### Example 8. Pharmacokinetics of Anti-HSA Antibodies in Mice

A pharmacokinetic study was conducted on the anti-HSA antibodies of the present disclosure (#7 _Hu_6-6his, #7 _Hu_7-6his, and #7_Hu_10-6his), a positive control antibody (ALB8-6his), and an isotype control antibody (Isotype, non-HSA-binding VHH molecule) in male CD-1 mice (Beijing Vital River Laboratory Animal Technology Co., Ltd.). The mice were divided into groups of three. On day 0, the mice were subjected to administration by tail vein injection at a dose of 4 mg/kg. Blood samples were collected before administration and at 5 min, 1 h, 7 h, 24 h, 48 h, 72 h, 96 h, 120 h and 144 h post-administration, then anticoagulated with EDTA, and centrifuged to prepare plasma for use in the subsequent pharmacokinetic assay.

PK assay: A 96-well microplate (Corning, Cat No. 9018) was coated with a 5 µg/mL anti-his antibody (Thermo, MAI-21315-1mg) at a volume of 100 µL/well and then left to stand overnight at 4 °C for 16-20 h. After the liquid was discarded, the plate was washed three times with PBST (pH 7.4, 0.05% Tween-20) buffer, and then a Casin Blocker (Thermo, 37528) blocking solution was added (300 µL/well). The mixture was incubated in an incubator at 37 °C for 2 h for blocking. After the blocking was completed, the blocking solution was discarded, and the plate was washed 3 times with PBST buffer. Then, mouse plasma samples from different time points, diluted appropriately with a diluent (Casin Blocker containing 1% CD1 mouse plasma), were added, and the plate was incubated at 37 °C for 1.5 h. After the incubation was completed, the reaction liquid in the plate was discarded, and the plate was washed 3 times with PBST. Then, 100 µL of an HRP-labeled rabbit anti-camel Cocktail VHH secondary antibody (Genescript, A02016, without BSA) (diluted at 1:5000) was added to each well, and the mixture was incubated at 37 °C for 1 h. After the plate was washed 3 times with PBST, 100 µL of a TMB chromogenic substrate was added for chromogenic reaction. The reaction was terminated with 1 M sulfuric acid, and the absorbance values at OD450 nm were taken using a SpectraMax M5 microplate reader. The concentrations of the VHH antibodies in the plasma samples were calculated based on a standard curve, and the results are shown in FIG. 3. The calculated concentrations were used in PK solver to determine the half-lives, and the results are shown in FIG. 4 and Table 8.

**Table 8. Mouse plasma half-lives of anti-HSA antibodies**

| Parameter (unit) | ALB8-6his | #7_Hu_6-6his | #7_Hu_7-6his | #7_Hu_10-6his | Isptype |
|---|---|---|---|---|---|
| | 5 mg/kg | 5 mg/kg | 5 mg/kg | 5 mg/kg | 5 mg/kg |
| T_{1/2} (h) | 7.16 | 9.0* | 10.7** | 9.83** | 1.12**** |
| AUC 0-inf_obs/dose (µg/mL*h/mg/kg) | 63 | 177 | 123 | 113 | 33.79 |
| Cl_obs (mL/(min*kg)) | 0.285 | 0.095 | 0.114 | 0.148 | 0.493 |

| | | | | | |
|---|---|---|---|---|---|
| (Note: *, p < 0.05; **, p < 0.01; ***, p < 0.0001; ****, p < 0.00001; all compared to the positive control ALB8-6his) | | | | | |

The results show that the anti-HSA antibodies #7_Hu_6-6his, #7_Hu_7-6his, and #7_Hu_10-6his of the present disclosure had similar pK curves, with half-lives within the range of mouse serum albumin's half-life and better than that of the positive control ALB8-6his antibody, and the lower clearance rates thereof were also lower than that of the ALB8-6his antibody.

### Example 9. Design and Preparation of Fusion Proteins

The relaxin A chain and B chain were linked by a linker to form a single-chain relaxin molecule, and meanwhile, in order to improve the half-life, the single-chain relaxin molecule was further fused to an anti-HSA single-domain antibody to form a fusion protein, the structural schematic diagram of which is shown in FIG. 5.
> Human RLN2 A chain
   QLYSALANKCCHVGCTKRSLARFC (SEQ ID NO: 31)
> Human RLN2 B chain (delB1)
   SWMEEVIKLCGRELVRAQIAICGMSTWS (SEQ ID NO: 32)
> Linker a
   GGGGQGGGGQGGGGQGGGGQGGGGQ (SEQ ID NO: 33)
> Linker b
   PGPAPGPAPGPAPGPAPGPAPGPAPGPAPGPA (SEQ ID NO: 34)
> Linker c
   PGPQPGPQPGPQPGPQPGPQPGPQPGPQPGPQ (SEQ ID NO: 35)
> Linker d
   GGGSGGSGGG (SEQ ID NO: 49)
> 0051 
EVQLLESGGGLVQPGGSLRLSCAASGFTFLTSCMGWFRQAPGKEREGVSSIYTVGGS
TFYTNSVKGRFTISRDNSKNTLYLQMNSLRAEDTAVYYCAAGSPARCRLRDPTTFDYW
GQGTLVTVSSGGGGQGGGGQGGGGQGGGGQGGGGQSWMEEVIKLCGRELVR
AQIAICGMSTWSGGGSGGSGGGQLYSALANKCCHVGCTKRSLARFC
(SEQ ID NO: 36)
> 0052 
EVQLLESGGGLVQPGGSLRLSCAASGFTFLTSCMGWFRQAPGKEREGVSSIYTVGGS
TFYTNSVKGRFTISRDNSKNTLYLQMNSLRAEDTAVYYCAAGSPARCRLRDPTTFDYW
GQGTLVTVSSPGPAPGPAPGPAPGPAPGPAPGPAPGPAPGPASWMEEVIKLCGRE
LVRAQIAICGMSTWSGGGSGGSGGGQLYSALANKCCHVGCTKRSLARFC
(SEQ ID NO: 37)
> 0053 
EVQLLESGGGLVQPGGSLRLSCAASGFTFLTSCMGWFRQAPGKEREGVSSIYTVGGS
TFYTNSVKGRFTISRDNSKNTLYLQMNSLRAEDTAVYYCAAGSPARCRLRDPTTFDYW
GQGTLVTVSSPGPQPGPQPGPQPGPQPGPQPGPQPGPQPGPQSWMEEVIKLCGR
ELVRAQIAICGMSTWSGGGSGGSGGGQLYSALANKCCHVGCTKRSLARFC
(SEQ ID NO: 38)
> 0062 
EVQLLESGGGLVQPGGSLRLSCAASGNTYLTSCMGWFRQAPGKEREGVSSIYTVGGS
TFYTNSVKGRFTISRDNSKNTLYLQMNSLRAEDTAVYYCAAGSPARCRLRDPTTFDYW
GQGTLVTVSSGGGGQGGGGQGGGGQGGGGQGGGGQSWMEEVIKLCGRELVR
AQIAICGMSTWSGGGSGGSGGGQLYSALANKCCHVGCTKRSLARFC
(SEQ ID NO: 39)
> 0063 
EVQLLESGGGLVQPGGSLRLSCAASGNTYLTSCMGWFRQAPGKEREGVASIYTVGGS
TFYTNSVKGRFTISRDNSKNTLYLQMNSLRAEDTAVYYCAAGSPARCRLRDPTTFDYW
GQGTLVTVSSGGGGQGGGGQGGGGQGGGGQGGGGQSWMEEVIKLCGRELVR
AQIAICGMSTWSGGGSGGSGGGQLYSALANKCCHVGCTKRSLARFCC
(SEQ ID NO: 40)
> 0064 
EVQLLESGGGLVQPGGSLRLSCAASGNTYLTSCMGWFRQAPGKEREGVSSIYTVGGS
TFYTNSVKGRFTISRDNSKNTLYLQMNSLQPEDTAVYYCAAGSPARCRLRDPTTFDYW
GQGTLVTVSSGGGGQGGGGQGGGGQGGGGQGGGGQSWMEEVIKLCGRELVR
AQIAICGMSTWSGGGSGGSGGGQLYSALANKCCHVGCTKRSLARFC
(SEQ ID NO: 41)
> 0065 
EVQLLESGGGLVQPGGSLRLSCAASGFTFLTSCMGWFRQAPGKEREGVSSIYTVGGS
TFYTNSVKGRFTISRDNSKNTLYLQMNSLRPEDTAVYYCAAGSPARCRLRDPTTFDYW
GQGTLVTVSSGGGGQGGGGQGGGGQGGGGQGGGGQSWMEEVIKLCGRELVR
AQIAICGMSTWSGGGSGGSGGGQLYSALANKCCHVGCTKRSLARFC
(SEQ ID NO: 42)
> 0066

EVQLLESGGGLVQPGGSLRLSCAASGNTYLTSCMGWFRQAPGKEREGVSSIYTVGGS
TFYTNSVKGRFTISRDNSKNTLYLQMNSLRPEDTAVYYCAAGSPARCRLRDPTTFDYW

GQGTLVTVSSGGGGQGGGGQGGGGQGGGGQGGGGQSWMEEVIKLCGRELVR
AQIAICGMSTWSGGGSGGSGGGQLYSALANKCCHVGCTKRSLARFC
(SEQ ID NO: 43)

The italicized parts indicate the anti-HSA single-domain antibody; the single underlined parts indicate linker 1; the double underlined parts indicate linker 2; the bold parts indicate the B chain of relaxin-2; the bold and italicized parts indicate the A chain of relaxin-2. Linker 1 can be selected from the group consisting of linkers a, b, and c, where a is a flexible linker, and b and c are rigid linkers.

The sequence of the positive control 0054 in this example was from Example 7 in WO2021022139A1.
> 0054

EVQLLESGGGLVQPGGSLRLSCAASGRYIDETAVAWFRQAPGKGREFVAGIGGGVDI
TYYADSVKGRFTISRDNSKNTLYLQMNSLRPEDTAVYYCAARPGRPLITSKVADLYPYW
GQGTLVTVSSGGGGQGGGGQGGGGQGGGGQGGGGQSWMEEVIKLCGRELVR
AQIAICGMSTWSGGGSGGSGGGQLYSALANKCCHVGCTKRSLARFC
(SEQ ID NO: 44).

### 1. Preparation of fusion proteins:

The DNA sequences encoding the fusion proteins (SEQ ID NOs: 36-44) were each codon-optimized, synthesized, and cloned into a pTT5 expression vector. Then, expiCHO (ThermoFisher, Cat No. A29127) cells were transiently transfected with the expression plasmid using the ExpiFectamine CHO Transfection Kit (ThermoFisher, Cat No. A29133) according to the kit manual. Following the "High protocol" in the manual, the day after transfection, the cells were placed in a shaker at 32 °C for suspension culture with shaking (5% CO₂, 110 rpm, 75% humidity). On day 10-12 after transfection, the supernatant of cell culture medium was collected and centrifuged at 4000 rpm for 20 min to collect a supernatant, which was then filtered using a 0.45 µm filter for later use.

### 2. Purification of fusion proteins:

(1) Fusion protein capture by A3 affinity chromatography: The cell culture supernatant expressing the fusion protein was subjected to high-speed centrifugation to collect the supernatant, which was then filtered using a 0.22 µm filter membrane. An A3 (JSR Life Sciences, Cat No. BP-AMS-A3-0100) affinity column was regenerated with 5 column volumes of 0.1 M NaOH (Sigma, Cat No. 71687), and then washed and equilibrated with 5 column volumes of 1× PBS (pH 7.4) (Sangon Biotech (Shanghai) Co., Ltd.: Cat No. E607016-0500). The filtered supernatant was loaded at a low flow rate to facilitate binding to the affinity column, with the flow rate controlled to ensure a retention time of about 1 min or longer. After the binding was completed, the chromatography column was washed with 5 column volumes of 1× PBS (pH 7.4), followed by 5 column volumes of 1× PBS (pH 7.4) and 0.8 M NaCl (Vetec, Cat No. V900058), and then 5 column volumes of 1× PBS (pH 7.4), until the UV absorbance fell back to baseline. The sample was eluted with a 0.1 M Glycine (pH 3.0) (Sigma, Cat No. 410225) buffer. The elution peaks were collected based on UV detection, and the eluted product was rapidly adjusted to pH 7-8 with 1 M Tris-HCl (pH 7.5) (Vetec, Cat No. V900483) and temporarily stored. For the eluted product, solution exchange could be performed by methods well known to those skilled in the art, such as ultrafiltration concentration using an ultrafiltration tube to exchange the solution to a desired buffer system, or size exclusion (e.g., G-25 desalination) to replace the solution with a desired buffer system.
(2) Fusion protein polishing purification by hydrophobic chromatography: Firstly, the sample was diluted with an equal volume of a buffer containing 20 mM Tris (pH 7.5) (Vetec, Cat No. V900483) and 1 M Na₂SO₄ (Sigma, Cat No. 239313). A Phenyl 650M (Toyopearl, Cat No. 0014478) hydrophobic chromatography column was regenerated with 5 column volumes of 0.5 M NaOH (Sigma, Cat No. 71687), and then washed and equilibrated with 10 column volumes of 20 mM Tris (pH 7.5) (Vetec, Cat No. V900483) and 0.5 M Na₂SO₄ (Sigma, Cat No. 239313). The diluted sample was loaded at a low flow rate to facilitate binding to the hydrophobic column, with the flow rate controlled to ensure a retention time of about 2 min or longer. After the binding was completed, the chromatography column was washed with 5 column volumes of 20 mM Tris (pH 7.5) (Vetec, Cat No. V900483) and 0.5 M Na₂SO₄ (Sigma, Cat No. 239313), until the UV absorbance fell back to baseline. 0-100% gradient elution was performed using an elution buffer containing 20 mM Tris (pH 7.5) (Vetec, Cat No. V900483); the elution peaks were collected based on SEC-HPLC (Column: TSKgel G3000SWXL, 5 µm, Cat No. 008541) purity determination. The yields of the fusion proteins after the two purification steps are shown in Table 9. For the eluted product, solution exchange could be performed by methods well known to those skilled in the art, such as ultrafiltration concentration using an ultrafiltration tube to exchange the solution to a desired buffer system, or size exclusion (e.g., G-25 desalination) to replace the solution with a desired buffer system.

**Table 9. Quality parameters of fusion proteins after two purification steps**

| Fusion protein | Expression yield (mg/L) | Purity (%) (SEC-HPLC) | Calculated molecular weight (Da) | Intact molecular weight (Da) (LC/MS) |
|---|---|---|---|---|
| 0051 | 150 | 99.7 | 21754.3 | 21755 |
| 0052 | 122 | 99.5 | 22551.6 | 22553 |
| 0053 | 156 | 99. | 23008 | 23009 |
| 0054 | 70 | 99.7 | 21797.5 | 21798 |

After the two purification steps, intact high-purity fusion proteins were obtained. Additionally, after transient transfection in expiCHO under the same conditions, the yields of the molecules 0051 and 0053 of the present disclosure were twice that of the positive control molecule 0054.

### Example 10. Assay for Affinity of Fusion Proteins for Serum Albumins from Different Species

The affinity of 0051 for human, monkey, rat, mouse, dog, pig, rabbit and bovine serum albumins was determined using a Biacore T200 (GE Healthcare) instrument. 0054 was used as a positive control. The experiment was performed using human albumin HSA (Sigma, Cat No. 126658), monkey albumin (Abcam, Cat No. ab184894), rat albumin (Abcam, Cat No. ab198656), mouse albumin (Abcam, Cat No. ab183228), rabbit albumin (Abcam, Cat No. ab188055), and bovine albumin (Sigma, Cat No. B2064).

### 1. Affinity assay using anti-VHH antibody-coupled chip

Coupling of an anti-VHH antibody (MonoRab^{™} Rabbit Anti-Camelid VHH Cocktail, Genescript, Cat No. A02014) to an S series CM5 sensor chip (GE, Cat No. 29-1049-88) was performed using an amino coupling kit (GE, Cat No. BR-1000-50). The antibody was diluted to 10 µg/mL with 10 mM sodium acetate (pH 4.5) (GE, Cat No. BR-1003-50). After the CM5 chip was activated with EDC/NHS, the diluted anti-VHH antibody was flowed over the chip to reach a surface response value of about 5000 RU, and then the chip was blocked with ethanolamine for later use.

The albumin from each species was serially diluted in a gradient with 1XHBS-EP (10 mM HEPES, pH 7.4, 150 mM NaCl, 3 mM EDTA, 0.05% Surfactant P20). The test fusion protein molecule was captured using the anti-VHH antibody-coupled chip, with a response value of about 30 RU. Then, different concentrations of serum albumin were flowed over the surface of the chip for 3 min at a flow rate of 50 µL/min, with a dissociation time of 1-10 min (the dissociation time varied depending on the species of albumin). After the end of each cycle, the chip was regenerated using 10 mM glycine-hydrochloric acid (pH 1.5) (GE, Cat No. BR-1003-54). The reaction signals were detected in real-time using Biacore T200 (GE) to obtain the association and dissociation curves, and the data obtained were fitted with a Langmuir 1:1 binding model using the BIAevaluation version 4.1 software at appropriate concentration points to obtain the affinity values. The results are shown in Table 10.

**Table 10. Affinity of fusion proteins for serum albumins from different species**

| Albumin type | Fusion protein | ka (1/Ms) | kd (1/s) | K_{D} (M) |
|---|---|---|---|---|
| | 0051 | 5.45E+04 | 1.10E-03 | 2.02E-08 |
| | 0052 | 8.99E+04 | 8.67E-04 | 9.65E-09 |
| | 0053 | 9.23E+04 | 7.88E-04 | 8.54E-09 |
| | 0062 | 1.45E+05 | 7.90E-04 | 5.44E-09 |
| HSA | 0063 | 1.47E+05 | 7.45E-04 | 5.07E-09 |
| | 0064 | 1.54E+05 | 8.60E-04 | 5.59E-09 |
| | 0065 | 8.23E+04 | 7.97E-04 | 9.68E-09 |
| | 0066 | 1.45E+05 | 7.78E-04 | 5.38E-09 |
| | 0054 | 3.57E+04 | 5.63E-04 | 1.58E-08 |
| Rat albumin | 0051 | 3.72E+04 | 5.81E-03 | 1.56E-07 |
| | 0052 | 4.13E+04 | 5.99E-03 | 1.45E-07 |
| | 0053 | 4.08E+04 | 5.91E-03 | 1.45E-07 |
| | 0062 | 9.15E+04 | 7.23E-03 | 7.91E-08 |
| | 0063 | 9.41E+04 | 6.48E-03 | 6.88E-08 |
| | 0064 | 9.89E+04 | 5.79E-03 | 5.85E-08 |
| | 0065 | 3.57E+04 | 5.67E-03 | 1.59E-07 |
| | 0066 | 8.34E+04 | 6.95E-03 | 8.34E-08 |
| | 0054 | 6.15E+04 | 1.13E-02 | 1.83E-07 |

The affinity of the different fusion proteins designed in the present disclosure for human, monkey, rat and bovine serum albumins was comparable to that of the positive control molecule 0054 (data not shown). Table 10 exemplarily presents the results for the binding of HSA to rat albumin.

### 2. Affinity assay using albumin-coupled chip

Coupling of serum albumins from different species to an S series CM5 sensor chip (GE, Cat. 29-1049-88) was performed using an amino coupling kit (GE, Cat No. BR-1000-50). The albumin from each species was diluted to 2 µg/mL. After the CM5 chip was activated with EDC/NHS, the diluted albumin was flowed over the chip to reach a surface response value of about 200 (180 to 230) RU, and then the chip was blocked with ethanolamine. The blank control channel also needed to be activated with EDC/NHS and blocked with ethanolamine.

The molecules 0051 and 0054 were serially diluted in a gradient using 1XHBS-EP (10 mM HEPES, pH 7.4, 150 mM NaCl, 3 mM EDTA, 0.05% Surfactant P20) at the following concentrations: 2000, 666.66, 222.22, 74.074, 24.69, 8.23, 2.74, 0.91, and 0.305 nM. The samples of 0051 and 0054 at different concentrations were separately flowed through the albumin-coupled and blank channels for 3 min at a flow rate of 50 µL/min, with a dissociation time of 4 min. After the end of each cycle, the chip was regenerated using 10 mM glycine-hydrochloric acid (pH 1.5) (GE, Cat. # BR-1003-54). The reaction signals were detected in real-time using Biacore T200 (GE) to obtain the association and dissociation curves, and the data obtained were fitted with a Langmuir 1:1 binding model using the BIAevaluation version 4.1 software at appropriate concentration points to obtain the affinity values.

**Table 11. Affinity for albumins from different species**

| Albumin type | Molecule | Ka (1/Ms) | Kd (1/s) | K_{D} (M) |
|---|---|---|---|---|
| HSA | 0051 | 4.91E+06 | 2.07E-04 | 4.22E-11 |
| | 0054 | 1.83E+06 | 1.81E-04 | 9.90E-11 |
| Rat albumin | 0051 | 2.25E+06 | 4.39E-03 | 1.95E-09 |
| | 0054 | 2.31E+06 | 2.46E-03 | 1.06E-09 |
| Mouse albumin | 0051 | 1.24E+06 | 4.71E-02 | 3.79E-08 |
| | 0054 | 1.97E+06 | 2.70E-03 | 1.37E-09 |

The affinity of the molecule 0051 for human, monkey and rat albumins was comparable to that of the positive control molecule 0054, but its affinity for mouse and dog albumins was weaker than that of the positive control molecule 0054 (data not shown). Table 11 exemplarily presents the results for HSA, rat albumin, and mouse albumin.

### Example 11. In Vitro Bioactivity of Fusion Proteins in Cells

### 1. Bioactivity of fusion proteins in THP-1 cells

THP-1 is a human peripheral blood monocyte (Procell, Cat No. CL-0233) that endogenously expresses the RXFP1 receptor. Relaxin binds to the receptor on THP-1 cells, inducing the production of cAMP in the THP-1 cells. Thus, in this example, the *in vitro* activity of the fusion proteins was assayed by detecting the amount of cAMP produced. In this example, the level of cAMP was assayed using a homogeneous time-resolved fluorescence (HTRF) cAMP-Gs Dynamic kit (Cisbio, Cat No. 62AM4PEB) based on the manufacturer's protocol. 0054, 800814 (derived from WO2015067113A1) and 0060 (WO2021255127A1, RELAX0023) were used as positive controls.
> A chain of 800814
   DLYSALANKCCHVGCTKRSLARFC (SEQ ID NO: 45)
> B chain of 800814
   DSWMEEVIKLCGRDLVRAQIAICGMSTWS (SEQ ID NO: 46)
> A chain of 0060 
DKTHTCPPCPAPEFEGGPSVFLFPPKPKDTLMISRTPEVTCVVVDVSHEDPEVKF
NWYVDGVEVHNAKTKPREEQYNSTYRVVSVLTVLHQDWLNGKEYKCKVSNK
ALPASIEKTISKAKGQPREPQVYTLPPCREEMTKNQVSLWCLVKGFYPSDIAVEW
ESNGQPENNYKTTPPVLDSDGSFFLYSKLTVDKSRWQQGNVFSCSVMHEALHN
HYTQKSLSLSPGGGGGSGGGGSGGGGSGGGGGSQLYSALANKCCHVGCTKRS
LARFC (SEQ ID NO: 47)
> B chain of 0060

DKTHTCPPCPAPEFEGGPSVFLFPPKPKDTLMISRTPEVTCVVVDVSHEDPEVKF
NWYVDGVEVHNAKTKPREEQYNSTYRVVSVLTVLHQDWLNGKEYKCKVSNK
ALPASIEKTISKAKGQPREPQVCTLPPSREEMTKNQVSLSCAVKGFYPSDIAVEW
ESNGQPENNYKTTPPVLDSDGSFFLVSKLTVDKSRWQQGNVFSCSVMHEALHN
HYTQKSLSLSPGGGGGSGGGGSGGGGSGGGGGSSWMEEVIKLCGRELVRAQIA
ICGMSTWS (SEQ ID NO: 48).

The experimental procedures were as follows: THP-1 cells were cultured at 37 °C with 5% CO₂ in a medium of RPMI1640 + 0.05 mM b-mercaptoethanol + 10% FBS + 1% penicillin-streptomycin. On the day of the experiment, THP-1 cells in the logarithmic growth phase were collected by centrifugation, resuspended in a serum-free medium, adjusted to a cell density of 4 × 10⁶ cells/mL, and then transferred to a 96-well assay plate (Cisbio, Cat No. 66PL96025) at a volume of 50 µL per well (20,000 cells/well). The test fusion protein samples and the positive controls 0054, 0060, and 800814 were subjected to 10-fold serial dilution using Stimulation Buffer 1 from the kit in separate U-bottom 96-well plates, and each transferred to the 96-well assay plate containing the THP-1 cells at a volume of 5 µL per well. The assay plate was then incubated at 37 °C with 5% CO₂ for 30 min. Cells without the addition of the test samples were used as a reference control. cAMP-d2 (acceptor fluorophore) and anti-cAMP-cryptat (donor fluorophore) were diluted with Lysis & Detection Buffer 1 from the kit. cAMP-d2 was added at a volume of 5 µL per well to all wells of the 96-well assay plate except the blank control wells, and then anti-cAMP-cryptat was added to all wells. The plate was incubated at room temperature in the dark for 1 h. Readings were then taken on Tecan (Infinite 200 PRO) at wavelengths of 620 nm and 665 nm. The signal intensity was expressed as the ratio of fluorescence intensity at 665 nm/fluorescence intensity at 620 nm × 10,000. Nonlinear fitting was performed using Graphpad Prism to plot cAMP concentration curves, obtaining the EC₅₀ values (nM) for cAMP production induced by the test samples and controls in THP-1 cells. The results are shown in Table 12, FIG. 6A, and FIG. 6B.

**Table 12. In vitro activity of fusion proteins (assayed in THP1 cells)**

| Molecule | Without HSA | 1% HSA |
|---|---|---|
| | EC₅₀ (nM) | EC₅₀ (nM) |
| 800814 | 0.2 | 0.32 |
| 0060 | 0.32 | 0.61 |
| 0054 | 10.3 | 15.82 |
| 0051 | 3.83 | 28.38 |
| 0052 | 5.22 | 21.52 |
| 0053 | 4.23 | 25.76 |
| 0062 | 4.02 | 23.06 |
| 0063 | 4.15 | 39.35 |
| 0064 | 8.5 | 19.75 |
| 0065 | 2.08 | 23.01 |
| 0066 | 3.49 | 23.47 |

The results show that the *in vitro* activity of the fusion proteins of the present disclosure was comparable to that of the positive control molecule 0054.

### 2. Bioactivity of fusion proteins in CHO-K1 cells overexpressing RXFP receptor

### (1) Preparation of CHO-K1 cells transiently transfected with RXFP1 and RXFP2 receptors

CHO-K1 cells (CCTCC, Cat No. GDC0018) were transiently transfected using Lipofectamine 2000 (Thermo Fisher, Cat No. 11668019). CHO-K1 cells were resuspended in a 6-well plate at a concentration of 6 × 10⁵ cells/well in advance. After 12 hours of culture at 37 °C with 5% CO₂, the original medium was removed, and 1.5 mL of serum-free Ham's F-12K (Kaighn's) medium (Gibco, Cat No. 21127030) was added. For each well of cells, the following procedures were performed: Two tubes were taken. In tube 1, 3 µg of the corresponding plasmid DNA (human, dog, cynomolgus monkey, rat or mouse RXFP1 receptor or human RXFP2 receptor DNA plasmid) was dissolved in 250 µL of Opti-MEM I reduced serum medium (Gibco, Cat No. 31985070). In tube 2, 3 µL of Lipofectamine 2000 reagent was dissolved in 250 µL of Opti-MEM I reduced serum medium. The tubes were incubated at room temperature for 5 min, and then the contents in tube 1 were added into tube 2. The mixture was gently mixed well, incubated at room temperature for 20 min, and then dropwise added to the 6-well plate (500 µL/well). After 6 hours of transfection, the medium was replaced with a complete medium, and the culture was continued for 24 h before proceeding with the subsequent experiment.

### (2) In vitro activation effect of fusion proteins on their receptors

In this example, the level of cAMP was assayed using a homogeneous time-resolved fluorescence (HTRF) cAMP-Gs Dynamic kit (Cisbio, Cat No. 62AM4PEB) based on the manufacturer's protocol. The specific procedures were as follows:

On the day of the experiment, the CHO-K1 cells transfected with the human, dog, cynomolgus monkey, rat or mouse RXFP1 receptor were collected by centrifugation, resuspended in a serum-free medium, adjusted to a cell density of 4 × 10⁶ cells/mL, and then transferred to a 96-well assay plate (Cisbio, Cat No. 66PL96025) at a volume of 50 µL per well (20,000 cells/well). The test sample 0051 and the positive controls 0054, 0060, and 800814 were subjected to 10-fold serial dilution using Stimulation Buffer 1 from the kit in separate U-bottom 96-well plates, and each transferred to the 96-well assay plate containing the THP1 cells at a volume of 5 µL per well. The assay plate was then incubated at 37 °C with 5% CO₂ for 30 min. Cells without the addition of the test sample were used as a reference control. cAMP-d2 (acceptor fluorophore) and anti-cAMP-cryptat (donor fluorophore) were diluted with Lysis & Detection Buffer 1 from the kit. cAMP-d2 was added at a volume of 5 µL per well to all wells of the 96-well assay plate except the blank control wells, and then anti-cAMP-cryptat was added to all wells. The plate was incubated at room temperature in the dark for 1 h. Readings were then taken on Tecan (Infinite 200 PRO) at wavelengths of 620 nm and 665 nm. The signal intensity was expressed as the ratio of fluorescence intensity at 665 nm/fluorescence intensity at 620 nm × 10,000. Nonlinear fitting was performed using Graphpad Prism to plot cAMP concentration curves, obtaining the EC₅₀ values (µM) for cAMP production induced by the fusion protein and controls in CHO-K1 cells with receptor overexpression. The results are shown in Table 13 and FIG. 6B.

**Table 13. In vitro activity of fusion proteins (CHO-K1 cells with receptor overexpression)**

| EC₅₀ (nM) | Human Rxfp1 | Dog Rxfp1 | Cynomolgus monkey Rxfp1 | Rat Rxfp1 | Mouse Rxfp1 | Human Rxfp2 |
|---|---|---|---|---|---|---|
| 800814 | 5.621 | 156.5 | 5.864 | 1.878 | 4.729 | 252.3 |
| 0054 | 25.65 | 3039 | 21.78 | 10.48 | 23.41 | 1347 |
| 0051 | 28.34 | 2207 | 25.12 | 21.11 | 21.67 | 1246 |
| 0060 | 39.46 | 1067 | 19.38 | 20.02 | 38.78 | No activity |

The results show that the *in vitro* activity of the molecule 0051 was comparable to that of the control molecules 0054 and 0060.

### Example 12. Pharmacokinetics of Fusion Proteins in Rats

Male SD rats aged 4-6 weeks and weighing 200-250 g were purchased from Beijing Vital River Laboratory Animal Technology Co., Ltd. During feeding, the rats had free access to food and water, in a 12/12-hour light/dark cycle, at a temperature of 16-26 °C, with a relative humidity of 40-70%. After the animals arrived at the facility, they were acclimatized for no less than 7 days. The day before the start of the experiment, the experimental animals were numbered and randomly divided into four groups of 3. On the day of the experiment, the four groups of experimental animals were injected intravenously or subcutaneously with the test drugs 0051 and 0054. The administration dose was 4mg/kg, and the injection volume was 5 mL/kg.

For administration by intravenous injection, blood samples were collected at the following time points: before administration and at 0.5, 1, 3, 6, 24, 48, 72, 96, 120, 168, 192 and 240 h post-administration. For administration by subcutaneous injection, blood samples were collected at the following time points: before administration and at 3, 6, 24, 48, 72, 96, 120, 168, 192 and 240 h post-administration. From each animal, 0.03 mL of whole blood was taken and anticoagulated with EDTA-K2. After collection, the blood was immediately centrifuged at 4500 rpm for 5 min, and the supernatant was taken, added into an EP tube, and stored at -20 °C. After the experiment was completed, it was transferred to -80 °C for storage.

The concentrations of the drugs 0051 and 0054 in plasma were measured using ELISA: A 96-well microplate (Corning, Cat No. 9018) was coated with a 0.4 µg/mL Anti-Relaxin 2/RLN2 antibody (Abcam, Cat No. EPR21832-15) at a volume of 100 µL/well and then left to stand overnight at 4 °C for 16-20 h. After the liquid was discarded, the plate was washed three times with PBST (pH 7.4, 0.05% Tween-20) buffer, and then a 4% bovine serum albumin solution was added (300 µL/well). The mixture was incubated at room temperature for 1 h for blocking. After the blocking was completed, the blocking solution was discarded, and the plate was washed 3 times with PBST buffer. Then, rat plasma samples from different time points, diluted appropriately with a diluent (Casin Blocker containing 1% rat plasma (Thermo, Cat No. 37528)), were added, and the mixture was incubated at 37 °C for 1 h. After the incubation was completed, the reaction liquid in the plate was discarded, and the plate was washed 3 times with PBST. Then, 100 µL of an HRP-labeled rabbit anti-camel Cocktail VHH secondary antibody (Genescript, Cat No. A02016, without BSA) (diluted at 1:5000) was added to each well, and the mixture was incubated at 37 °C for 1 h. After the plate was washed 3 times with PBST, 100 µL of a TMB (Sigma, Cat No. T0440) chromogenic substrate was added for chromogenic reaction. The reaction was terminated with 1 M sulfuric acid, and the absorbance values at OD450 nm were taken using a SpectraMax M5 microplate reader.

The data results were analyzed using the Graphpad Prism software, and the concentrations of the molecules 0051 and 0054 in the plasma samples were calculated based on a standard curve. The results for the rats are shown in FIG. 7A. The calculated concentrations were used in PK solver to determine the half-lives, and the results are shown in Table 14. Additionally, we used a similar method to examine the pharmacokinetics in mice, and the results show that, as shown in FIG. 7B, the AUC exposure and t_{1/2} of the molecule 0051 were comparable to those of the positive control molecule 0054.

**Table 14. Pharmacokinetic parameters in rats**

| PK parameter | Rat | | | |
|---|---|---|---|---|
| | IV | | SC | |
| | 0051 | 0054 | 0051 | 0054 |
| AUC (0-t) (h*nmol/L) | 153397 | 327981 | 78268 | 97492 |
| AUC (inf) (h*nmol/L) | 157174 | 331235 | 83492 | 100424 |
| CLp_(mL/min/kg) | 0.02 | 0.01 | / | / |
| T1/2 (h) | 44.3 | 40.4 | 54.4 | 45 |
| AUClast/dose | 767 | 1640 | / | / |
| MRT (inf)_(h) | 57.5 | 34.7 | 96 | 77 |
| MRTlast | 51.4 | 32 | 81 | 70 |
| Vdss_(L/kg) | 0.07 | 0.02 | / | / |
| Tmax | / | / | 48 | 32 |
| Cmax | / | / | 948 | 1536 |
| Bioavailability% | / | / | 53.1 | 30.3 |

The PK results in rats show that after IV administration, the AUC of 0054 was approximately twice that of 0051, with little difference in the t1/2 of the 2 molecules; after SC administration, the bioavailability of 0051 was higher than that of 0054. Compared to the molecule 0054, 0051 had a larger apparent volume of distribution (Vdss), indicating that the molecule 0051 had better tissue and cell barrier permeability. Furthermore, 0051 had a longer apparent mean residence time (MRT), suggesting it might exert its pharmacological effects for a longer duration *in vivo.* Additionally, after administration by subcutaneous (SC) injection, the bioavailability (Bioavailability%) of 0051 was significantly higher than that of 0054. Thus, 0051 had better pharmacokinetic properties.

### Example 13. Effects of Fusion Proteins on Myocardial Hypertrophy in Mice

To establish a myocardial hypertrophy model in C57BL/6J mice (Charles River Laboratories), isoproterenol (ISO, Sigma, Cat. # I5627) was administered by fusion using a subcutaneously implanted micro-osmotic pump (Alzet 1002). ISO was dissolved in a PBS buffer (Servicebio, Cat. # G4202) containing 0.002% sodium ascorbate (Sigma, Cat. # 11140) to achieve a concentration of 146 mg/mL (calculated based on the average mouse body weight of 25.8 g). The infusion concentration was 15 mg/kg/d, and the infusion was performed continuously for 14 days to establish the pathological model. On day 14, the osmotic pump was removed, and the model control group 1 was euthanized for sample collection, while the remaining mice were randomly divided into groups and began to be subcutaneously injected with the fusion proteins or the control molecules. The administration dose and grouping are shown in Table 15.

**Table 15. Administration dose and grouping information**

| Group | Number | Establishment of myocardial hypertrophy model | Administration dose |
|---|---|---|---|
| Sham surgery | 5 | Only received sham surgery | - |
| Blank control | 5 | Blank control (PBS containing 0.002% ascorbic acid) | PBS (S.C, Q3d) |
| Model control 1 | 5 | 15 mg/kg/d ISO in blank control | - |
| Model control 2 | 16 | 15 mg/kg/d ISO in blank control | PBS (S.C, Q3d) |
| 0051 at low dose | 17 | 15 mg/kg/d ISO in blank control | 2.5 mg/kg (S.C, Q3d) |
| 0051 at high dose | 12 | 15 mg/kg/d ISO in blank control | 7.4 mg/kg (S.C, Q3d) |
| 0054 at low dose | 5 | 15 mg/kg/d ISO in blank control | 2.5 mg/kg (S.C, Q3d) |
| 0054 at high dose | 12 | 15 mg/kg/d ISO in blank control | 7.4 mg/kg (S.C, Q3d) |
| 0060 at high dose | 12 | 15 mg/kg/d ISO in blank control | 20 mg/kg (S.C, Q7d) |

At the end of the study, after a 6-hour fast, the mice were weighed and then euthanized to measure the heart weight and tibia length. The condition of myocardial hypertrophy was judged by the ratio of heart weight to tibia length. The results are shown in FIG. 8 and Table 16.

**Table 16. Ratios of heart weight to tibia length in mouse ISO myocardial hypertrophy model**

| Group | Mean value | P value |
|---|---|---|
| Sham surgery | 7.38 | / |
| Blank control | 7.34 | / |
| Model control 1 | 8.98 | 0.0129 |
| Model control 2 | 7.34 | / |
| 0051 at low dose | 6.83 | 0.0345 |
| 0051 at high dose | 6.63 | 0.0078 |
| 0054 at low dose | 7.36 | 0.9548 |
| 0054 at high dose | 6.86 | 0.05 |
| 0060 at high dose | 6.87 | 0.0563 |

The above metric data are expressed as mean ± standard deviation (mean ± SD). Comparison between two groups was using the Student t test; * p < 0.05, ** p < 0.01 vs. model control group 2; # p < 0.05 vs. blank control group.

The infusion of ISO in mice could significantly induce myocardial hypertrophy, and the administration of the fusion protein of the present disclosure for treatment could reverse ISO-induced myocardial hypertrophy to the baseline level, requiring a significantly lower dose as compared to the control 0060.

## Claims

1. A fusion protein, comprising a serum albumin-binding domain and relaxin or an analog thereof,
the serum albumin-binding domain comprising an immunoglobulin single variable domain, wherein the immunoglobulin single variable domain comprises a CDR1, a CDR2, and a CDR3 selected from any one of 1)-3):
1) a CDR1, a CDR2, and a CDR3 from the amino acid sequence set forth in any one of SEQ ID NOs: 6 and 18-27;
2) a CDR1, a CDR2, and a CDR3 from the amino acid sequence set forth in SEQ ID NO: 7; and
3) a CDR1, a CDR2, and a CDR3 of the amino acid sequences set forth in SEQ ID NOs: 64, 58, and 59, respectively;
wherein the CDRs are defined according to the Kabat, IMGT, Chothia, AbM, or Contact numbering scheme;
preferably, the CDR1, the CDR2, and the CDR3 are set forth in SEQ ID NOs: 8, 9, and 10, or SEQ ID NOs: 11, 12, and 13, respectively.

2. The fusion protein according to claim 1, wherein the immunoglobulin single variable domain is engineered by any one selected from the group consisting of the following: camelization or humanization, affinity maturation, T cell epitope removal, reduction of antibody deamidation, reduction of antibody aggregation, reduction of antibody isomerization, and a combination thereof;
preferably, a framework region template of a human germline gene for the humanization engineering is derived from IGHV3-23.

3. The fusion protein according to any one of claims 1-2, wherein the immunoglobulin single variable domain comprises an amino acid mutation at at least one of the following positions: positions 20, 23, 27, 29, 30, 37, 44, 45, 47, 49, 74, 78, 83, and 84;
the positions are based on the EU numbering scheme;
preferably, the immunoglobulin single variable domain comprises at least one amino acid mutation selected from the group consisting of the following: 20V, 23V, 27N, 29Y, 30L, 37F, 44E, 45R, 47G, 49A, 74A, 78V, 83Q, and 84P;
more preferably, the immunoglobulin single variable domain comprises a combination of amino acid mutations selected from any one of the following:
27N, 29Y, 30L, 37F, 44E, 45R, and 47G;
20V, 27N, 29Y, 30L, 37F, 44E, 45R, and 47G;
23V, 27N, 29Y, 30L, 37F, 44E, 45R, and 47G;
27N, 29Y, 30L, 37F, 44E, 45R, 47G, and 74A;
27N, 29Y, 30L, 37F, 44E, 45R, 47G, and 78V;
27N, 29Y, 30L, 37F, 44E, 45R, 47G, and 49A;
27N, 29Y, 30L, 37F, 44E, 45R, 47G, 83Q, and 84P;
27N, 29Y, 30L, 37F, 44E, 45R, 47G, and 84P;
30L, 37F, 44E, 45R, 47G, and 84P; or
30L, 37F, 44E, 45R, and 47G.

4. The fusion protein according to any one of the preceding claims, wherein the immunoglobulin single variable domain
comprises the amino acid sequence set forth in any one of SEQ ID NOs: 6 and 18-27 or an amino acid sequence having at least 90% sequence identity thereto, or
comprises the amino acid sequence set forth in SEQ ID NO: 7 or an amino acid sequence having at least 90% sequence identity thereto.

5. The fusion protein according to any one of the preceding claims, wherein the immunoglobulin single variable domain is a VHH.

6. The fusion protein according to any one of the preceding claims, wherein the relaxin or the analog thereof comprises an A chain and a B chain, wherein the A chain has the amino acid sequence set forth in any one of SEQ ID NOs: 31, 50, 52, 54, and 56 or an amino acid sequence having at least 90% sequence identity thereto, and the B chain has the amino acid sequence set forth in any one of SEQ ID NOs: 32, 51, 53, 55, and 57 or an amino acid sequence having at least 90% sequence identity thereto;
preferably, the A chain and the B chain are selected from any one of the following 1)-7):
1) an A chain set forth in SEQ ID NO: 31 and a B chain set forth in SEQ ID NO: 32;
2) an A chain set forth in SEQ ID NO: 50 and a B chain set forth in SEQ ID NO: 51;
3) an A chain set forth in SEQ ID NO: 52 and a B chain set forth in SEQ ID NO: 53;
4) an A chain set forth in SEQ ID NO: 54 and a B chain set forth in SEQ ID NO: 55;
5) an A chain set forth in SEQ ID NO: 31 and a B chain set forth in SEQ ID NO: 53;
6) an A chain set forth in SEQ ID NO: 52 and a B chain set forth in SEQ ID NO: 32; and
7) an A chain set forth in SEQ ID NO: 56 and a B chain set forth in SEQ ID NO: 57.

7. The fusion protein according to claim 6, comprising a polypeptide shown in any one of or any combination of formula (I) to formula (IV):
B-L₁-A-L₂-VHH formula (I)
A-L₁-B-L₂-VHH formula (II)
VHH-L₂-B-L₁-A formula (III)
VHH-L₁-A-L₁-B formula (IV)
wherein
A is the A chain of the relaxin or the analog thereof;
B is the B chain of the relaxin or the analog thereof;
L₁ and L₂ are linkers;
L₁ and L₂ are each independently present or absent.

8. The fusion protein according to claim 7, wherein
Li comprises an amino acid sequence selected from the group consisting of (GGGGQ)n, (GGGQ)n, (PGPQ)n, (PGPA)n, and (G_{X}S_{Y})_{Z}, wherein n is selected from the group consisting of integers of 1-10, and X, Y, and Z are independently selected from the group consisting of integers of 1-10;
L₂ comprises an amino acid sequence set forth in any one selected from the group consisting of (G_{X}S_{Y})_{Z}, KR, and SEQ ID NOs: 60-63 and 65-66, wherein X, Y, and Z are independently selected from the group consisting of integers of 1-10;
preferably, Li comprises the amino acid sequence set forth in any one of SEQ ID NOs: 33-35;
preferably, L₂ comprises the amino acid sequence set forth in SEQ ID NO: 49.

9. The fusion protein according to any one of the preceding claims, comprising the amino acid sequence set forth in any one of SEQ ID NOs: 36-43 or an amino acid sequence having at least 90% sequence identity thereto.

10. The fusion protein according to any one of the preceding claims, being a single-chain protein, a dimeric protein, or a multimeric protein.

11. A serum albumin-binding molecule, comprising an immunoglobulin single variable domain, the immunoglobulin single variable domain comprising a CDR1, a CDR2, and a CDR3 selected from any one of 1)-3):
1) a CDR1, a CDR2, and a CDR3 from the amino acid sequence set forth in any one of SEQ ID NOs: 6 and 18-27;
2) a CDR1, a CDR2, and a CDR3 from the amino acid sequence set forth in SEQ ID NO: 7; and
3) a CDR1, a CDR2, and a CDR3 of the amino acid sequences set forth in SEQ ID NOs: 64, 58, and 59, respectively;
wherein the CDRs are defined according to the Kabat, IMGT, Chothia, AbM, or Contact numbering scheme;
preferably, the CDR1, the CDR2, and the CDR3 are set forth in SEQ ID NOs: 8, 9, and 10, or SEQ ID NOs: 11, 12, and 13, respectively.

12. The serum albumin-binding molecule according to claim 11, wherein the immunoglobulin single variable domain is engineered by any one selected from the group consisting of the following: camelization or humanization, affinity maturation, T cell epitope removal, reduction of antibody deamidation, reduction of antibody aggregation, reduction of antibody isomerization, and a combination thereof;
preferably, a framework region template of a human germline gene for the humanization engineering is derived from IGHV3-23.

13. The serum albumin-binding molecule according to any one of claims 11-12, wherein the immunoglobulin single variable domain comprises an amino acid mutation at at least one of the following positions: positions 20, 23, 27, 29, 30, 37, 44, 45, 47, 49, 74, 78, 83, and 84;
the positions are based on the EU numbering scheme;
preferably, the immunoglobulin single variable domain comprises at least one amino acid mutation selected from the group consisting of the following: 20V, 23V, 27N, 29Y, 30L, 37F, 44E, 45R, 47G, 49A, 74A, 78V, 83Q, and 84P;
more preferably, the immunoglobulin single variable domain comprises a combination of amino acid mutations selected from any one of the following:
27N, 29Y, 30L, 37F, 44E, 45R, and 47G;
20V, 27N, 29Y, 30L, 37F, 44E, 45R, and 47G;
23V, 27N, 29Y, 30L, 37F, 44E, 45R, and 47G;
27N, 29Y, 30L, 37F, 44E, 45R, 47G, and 74A;
27N, 29Y, 30L, 37F, 44E, 45R, 47G, and 78V;
27N, 29Y, 30L, 37F, 44E, 45R, 47G, and 49A;
27N, 29Y, 30L, 37F, 44E, 45R, 47G, 83Q, and 84P;
27N, 29Y, 30L, 37F, 44E, 45R, 47G, and 84P;
30L, 37F, 44E, 45R, 47G, and 84P; or
30L, 37F, 44E, 45R, and 47G.

14. The serum albumin-binding molecule according to any one of claims 11-13, wherein the immunoglobulin single variable domain
comprises the amino acid sequence set forth in any one of SEQ ID NOs: 6 and 18-27 or an amino acid sequence having at least 90% sequence identity thereto, or
comprises the amino acid sequence set forth in SEQ ID NO: 7 or an amino acid sequence having at least 90% sequence identity thereto.

15. The serum albumin-binding molecule according to any one of claims 11-14, wherein the immunoglobulin single variable domain is a VHH.

16. The serum albumin-binding molecule according to any one of claims 11-15, further comprising a human immunoglobulin Fc region or a histidine tag, wherein
preferably, the Fc region is an Fc region of human IgG1, IgG2, IgG3, or IgG4.

17. The serum albumin-binding molecule according to any one of claims 11-16, further comprising one or more therapeutic agents or diagnostic agents, wherein
preferably, the therapeutic agent or the diagnostic agent is covalently linked or fused to the immunoglobulin single variable domain;
preferably, the therapeutic agent or the diagnostic agent is selected from any one of the following: a therapeutic or diagnostic protein, polypeptide, nucleic acid or small-molecule compound.

18. The serum albumin-binding molecule according to any one of claims 11-17, having an activity selected from at least one of the following:
(a) binding to human serum albumin with a K_{D} value of ≤ 1 × 10⁻⁷ M, preferably ≤ 1 × 10⁻⁸ M;
(b) no detectable blocking of the binding of serum albumin to FcRn; and
(c) when comprising the therapeutic agent or the diagnostic agent, extending the plasma half-life of the therapeutic agent or the diagnostic agent.

19. A polynucleotide, encoding the fusion protein according to any one of claims 1-10 or the serum albumin-binding molecule according to any one of claims 11-18.

20. A vector, comprising or expressing the polynucleotide according to claim 19.

21. A host cell, comprising or expressing the vector according to claim 20.

22. A method for producing or preparing the fusion protein according to any one of claims 1-10 or the serum albumin-binding molecule according to any one of claims 11-18, comprising the steps of:
culturing the host cell according to claim 22;
collecting the fusion protein or the serum albumin-binding molecule; and
optionally, purifying and/or modifying the fusion protein or the serum albumin-binding molecule.

23. A method for extending the plasma half-life of a therapeutic agent or a diagnostic agent, comprising:
linking or fusing the immunoglobulin single variable domain defined in any one of claims 11-18 to the therapeutic agent or the diagnostic agent.

24. A pharmaceutical composition, comprising
the fusion protein according to any one of claims 1-10, the serum albumin-binding molecule according to any one of claims 11-18, the polynucleotide according to claim 19, or the vector according to claim 20,
preferably, further comprising one or more pharmaceutically acceptable carriers, diluents, buffers or excipients.

25. Use of the fusion protein according to any one of claims 1-10 in the preparation of a medicament for treating and preventing a disease, wherein
preferably, the disease is a fibrotic disease or a cardiovascular disease;
more preferably, the cardiovascular disease is heart failure or myocardial hypertrophy.

26. Use of the serum albumin-binding molecule according to any one of claims 11-18 in the preparation of a medicament for diagnosing, treating, and preventing a disease.
